# EUROPEAN PATENT APPLICATION

(11) **EP 4 477 751 A1**
(43) Date of publication of application: **18.12.2024**
(21) Application number: 23179680.6
(22) Date of filing: 16.06.2023
(51) Int. Cl.: C12N 15/10, C12Q 1/6806

(54) **BODY FLUID STABILIZATION AND PROCESSING**

(71) Applicant: QIAGEN GmbH, 40724 Hilden (DE)
(72) Inventor: OELMÜLLER, Uwe Dr., 40724 Hilden (DE); MANCARELLA, Daniela Dr., 40724 Hilden (DE); GRÖLZ, Daniel Dr., 40724 Hilden (DE)
(74) Representative: König Szynka Tilmann von Renesse Patentanwälte Partnerschaft mbB Düsseldorf

(57) **Abstract**

The present disclosure provides methods and kits for stabilizing extracellular nucleic acids in a cell-depleted biological sample obtained from a fluid cell-containing biological sample, which preferably is a urine sample.

## Description

### FIELD OF THE INVENTION

The present invention provides methods for stabilizing extracellular nucleic acids in a cell-depleted biological sample that was obtained from a fluid cell-containing biological sample, which preferably is a body fluid such as a urine sample. Also provided are advantageous kits and uses.

### BACKGROUND OF THE INVENTION

Most of the nucleic acids in the body are located within cells, but a small amount of nucleic acids is found extracellular in human body fluids, such as blood or urine. These nucleic acids are commonly referred to as extracellular or cell-free nucleic acids (e.g. cell-free DNA (cfDNA) and cell-free RNA (cfRNA)). Extracellular nucleic acids are also comprised in extracelluar vesicles, such as exosomes which in particular contain exosomal RNA. Analysis of extracellular nucleic acid levels as well as the detection of alterations of extracellular nucleic acids, such as mutations or DNA methylation changes, are of particular interest with regard to screening, prognosis and monitoring of medical conditions, including malignancies, inflammations and infectious processes.

Fast degradation and fragmentation of extracellular nucleic acids in body fluids such as urine and also release of cellular nucleic acids from contained cells, which dilutes and contaminates the initial cell-free nucleic acids after sample collection, challenge the use of cell-free nucleic acids as analytes. This is a particular challenge for the processing of urine samples. Post-collection microbial growth in the urine specimen can further enhance the degradation of nucleic acids. These alterations can render the results of diagnostics or research unreliable or even impossible because the subsequent analytical assay will not determine the situation in the body upon collection but instead determines an artificial profile that is generated after sample collection and prior to analysis. Furthermore, several labor-intensive steps, including cell separation by centrifugation, are necessary for isolating extracellular nucleic acids from a body sample such as urine. These steps require open handling of e.g. potentially infectious urine samples putting the health of the operator at risk and increasing the risk that the sample is contaminated with bacteria by the environment.

To prevent or reduce the above-mentioned problems, a fast, convenient and efficient stabilization of body fluids such as urine is necessary with reduced labor input and without chemical modification of the extracellular nucleic acids that potentially affects downstream analysis. Tubes comprising stabilizing solutions for extracellular nucleic acids from body fluids such as blood are described in the art (see e.g. WO2013/045457, WO2013/045458, WO2014/146780, WO2014/146782, WO2017/085321 and Streck Cell-Free DNA BCT Cat. No. 218997). Evacuated tubes in combination with urine collection cups (e.g. BD Urinalysis cup kit, Cat. No. 364981) are also used in practice, e.g. in clinics. However, either tubes without any stabilization for clinical chemistry analysis (e.g. BD Vacutainer, Cat. No. 364938) or with a stabilization for microbiological analysis and clinical chemistry are used (e.g. BD Vacutainer, Cat. No. 364955). Urine stabilization reagents for nucleic acids are also commercially available (Smart tube US20090155838A1; WO2017201612A1; Norgen, Cat. No. NC0930383; Zymo urine conditioning buffer, D3061-1-8, D3061-1-140). However, these reagents are generally intended for nucleic acid stabilization in urine but not specifically for extracellular nucleic acids. Although, preservatives for extracellular nucleic acid stabilization in urine can be found on the market, these stabilizers are usually not included in evacuated tubes. The stabilization cannot be realized as fast, convenient and safe as possible and laboratory personnel is necessary. The mixing ratio of urine and preservative is variable. Furthermore, some of these stabilizing reagents include crosslinking agents that could interfere with downstream analysis (see e.g. WO2018222709A4, WO2008155549A2, WO2016130543A1, EP3662080A1, US20160257995A1).

A home collection and stabilization system for extracellular nucleic acids in urine with the stabilizer included in the lid of a urine collection cup is e.g. known from WO2018057938A1. As soon as the lid of the collection cup is closed, the stabilizer will be united with the urine. However, the mixing ratio of urine and stabilizer varies and is dependent on the volume of urine donated. A urine collection system is furthermore disclosed in US 11,622,752.

An instant preservation of urine cell-free nucleic acids is also described in WO2020141187A1 where an evacuated tube (vacutainer) containing a stabilizing solution is coupled to a urine collection container with a cap containing a needle. The disclosed stabilization solution advantageously does not require the use of a cross-linking agent. However, the system disclosed in WO2020141187 needs improvement, in particular with respect to the processing of the stabilized urine samples.

Thus, the prior art technologies do not fully meet the requirements for instant and effective stabilization of extracellular nucleic acid profiles in body fluids such as urine. They either do not provide immediate stabilization without the need for clinical personnel or laboratory facilities or they need improvement with respect to the stabilization of extracellular nucleic acids. Furthermore, prior art methods are often labor-intensive.

It is thus an object of the present invention to avoid one or more drawbacks of the prior art and provide improved technologies, such as methods and kits, for stabilizing extracellular nucleic acids contained in fluid cell-containing biological sample, in particular a body fluid such as a urine sample, for subsequent isolation of the extracellular nucleic acids.

It is furthermore an object to provide an easy-to-use home collection or point-of-care kit for instant extracellular nucleic acid stabilization in urine without the need for clinical personnel or laboratory facilities. In particular, there is a need for a home and point-of-care collection system that achieves an instant and improved stabilization of the contained extracellular nucleic acids that does not require trained lab personnel. Point-of-care in this context includes but is not limited to doctors' offices, bed-site in clinics, pharmacies, and collection centers.

### SUMMARY OF THE INVENTION

The present invention provides improved technologies for stabilizing extracellular nucleic acids comprised in fluid cell-containing biological samples, in particular body fluids such as urine samples. A core feature of the present invention is the use of a separation device, such as a filter element, that allows to directly remove cells from the collected cell-containing biological sample with minimal effort, thereby providing a cell-depleted biological sample that is then stabilized for preserving the contained extracellular nucleic acids in a reception device that is suitable for transportation and/or storage. According to the invention, the cells are directly removed during the transfer of the cell-containing biological sample into the reception device (e.g. an evacuated tube) that comprises a stabilizing composition for stabilizing extracellular nucleic acids. The direct removal of cells is advantageous, because it reduces the risk of contaminating the extracellular nucleic acids by cellular nucleic acids that can be released from contained cells. The instant stabilization of the obtained cell-depleted biological sample by the stabilizing composition achieves an effective reduction or elimination of extracellular nucleic acid degradation. The stabilized cell-depleted biological sample is advantageously suitable for storage and shipping. The stabilized cell-depleted biological sample can be further processed, e.g. to purify extracellular nucleic acids or other components.

The present invention is particularly suitable for processing and stabilizing urine samples as cell-containing biological samples. As is moreover apparent from the below detailed description, the invention is suitable for use at home and point-of-care collection and stabilization and does not require trained personnel.

According to a first aspect, a method is provided for stabilizing extracellular nucleic acids contained in a fluid cell-depleted biological sample obtained from a fluid cell-containing biological sample, which preferably is a urine sample, said method comprising
(a) obtaining a fluid cell-containing biological sample in a collection device;
(b) establishing a connection to a reception device which comprises a stabilizing composition suitable for stabilizing extracellular nucleic acids;
(c) transferring the obtained cell-containing biological sample through a separation device, thereby separating cells and providing a cell-depleted biological sample and contacting the cell-depleted biological sample with the stabilizing composition comprised in the reception device, wherein preferably, the transfer is driven by the reception device.

According to a second aspect, a method is provided for isolating extracellular nucleic acids from a stabilized cell-depleted biological sample, the method comprising
- stabilizing extracellular nucleic acids in a cell-depleted biological sample obtained from a fluid cell-containing biological sample using the method according to the first aspect; and
- isolating extracellular nucleic acids from the stabilized cell-depleted biological sample.

According to a third aspect, a kit is provided for stabilizing extracellular nucleic acids in a cell-depleted biological sample obtained from a fluid cell-containing biological sample, which preferably is a urine sample, said kit comprising
(a) a collection device;
(b) a reception device comprising a stabilizing composition suitable for stabilizing extracellular nucleic acids comprised in the cell-depleted biological sample; and
(c) a separation device that comprises or consists of a separation element that is capable of retaining cells contained in the collected biological sample.

The kit may further comprise
(d) a closing means for closing the collection device; and/or
(e) a transfer device for assisting the transfer of fluids from the collection device into the reception device.

According to a fourth aspect, the present invention is directed to the use of a kit according to the third aspect in a method according to the first and/or second aspect.

According to a fifth aspect, the present invention is directed to the use of a separation device that comprises or consists of a separation element that is capable of retaining cells in a method for stabilizing extracellular nucleic acids contained in a fluid cell-depleted biological sample obtained from a fluid cell-containing biological sample, wherein stabilization of the cell-depleted biological sample is performed in a reception device that comprises a stabilizing composition, and wherein said method comprises transferring the cell-containing biological sample through the separation device, thereby separating cells and providing a cell-depleted biological sample and contacting the provided cell-depleted biological sample with the stabilizing composition comprised in the reception device, wherein the transfer is driven by the reception device.

As is apparent from the detailed description, the present invention provides improved methods and kits for processing and stabilizing a fluid cell-containing biological sample, in particular a urine sample, compared to prior art methods. It provides advantageous methods, kits and uses that effectively reduce or prevent the release of nucleic acids and nucleases from cells by separating the cells present in the cell-containing biological sample rapidly after specimen collection with minimal effort and in embodiments in an automated manner. Separation of cells and optionally further undesired components is advantageously achieved during the transfer of the cell-containing biological sample from the collection device, such as a urine collection cup, into the reception device, which preferably is an evacuated tube. The early separation of cells prevents contamination and dilution of target analytes, i.e. extracellular nucleic acids, in the cell-depleted biological sample by cellular nucleic acids. The extracellular nucleic acids comprised in the obtained cell-depleted biological sample are directly stabilized with a stabilizing composition that is comprised in a reception device that is also suitable for storage and transportation of the stabilized sample. As a contamination and dilution of the target analytes, preferably extracellular nucleic acids (e.g. cfDNA, cfRNA), is significantly reduced and extracellular nucleic acids are effectively stabilized in the cell-depleted biological sample, more reliable results can be obtained when the target extracellular nucleic acids are analysed, such as e.g. quantified.

The technology of the present invention allows the convenient and safe collection of the fluid cell-containing biological sample and mixing with the stabilizing composition as fast as possible after donation. In particular, a simpler, faster, and more convenient processing of fluid cell-containing biological samples is provided as cells are separated with minimal effort and in an automated manner during transfer into the reception device. Furthermore, the safety is increased since no open handling of the potentially infectious sample is required. This also allows sterile handling of the sample during processing and prevents contamination by the environment. In addition, less equipment and less processing steps are required due to simultaneous cell separation (no centrifugation required) and transfer of the sample into the reception device. Hence, the methods and kits of the present invention increase the reliability of results obtained using the extracellular nucleic acids. The technology is suitable for home collection and point-of-care.

In preferred embodiments, the present invention provides a urine collection, stabilization, transport and temporary storage device system combining an evacuated reception device, such as an evacuated plastic tube with a pierceable cap/lid (e.g. conventional evacuated tube usually used for blood collection, such as BD Vacutainer), that includes a stabilizing solution for extracellular nucleic acids from a fluid cell-containing biological sample, such as urine, and that can be coupled to a collection device, e.g. a urine collection cup, with a separation device, preferably a filter, that can be located e.g. within the collection device, between the collection device and the reception device (e.g. in a transfer device) or in the reception device itself. During passage into the reception device, cells are separated by the separation device. An instant preservation of urine cell-free nucleic acids is provided by the methods and kits of the present invention allowing a simple, fast and safe cell separation and stabilization workflow that can be conducted by the patient, e.g. at the doctor's office, in a clinic or at home, without the need for clinical personnel or laboratory facilities and equipment (e.g. centrifuge).

Other objects, features, advantages, and aspects of the present application will become apparent to those skilled in the art from the following description and appended claims. It should be understood, however, that the following description, appended claims, and specific examples, while indicating preferred embodiments of the application, are given by way of illustration only.

### BRIEF DESCRIPTION OF THE FIGURES

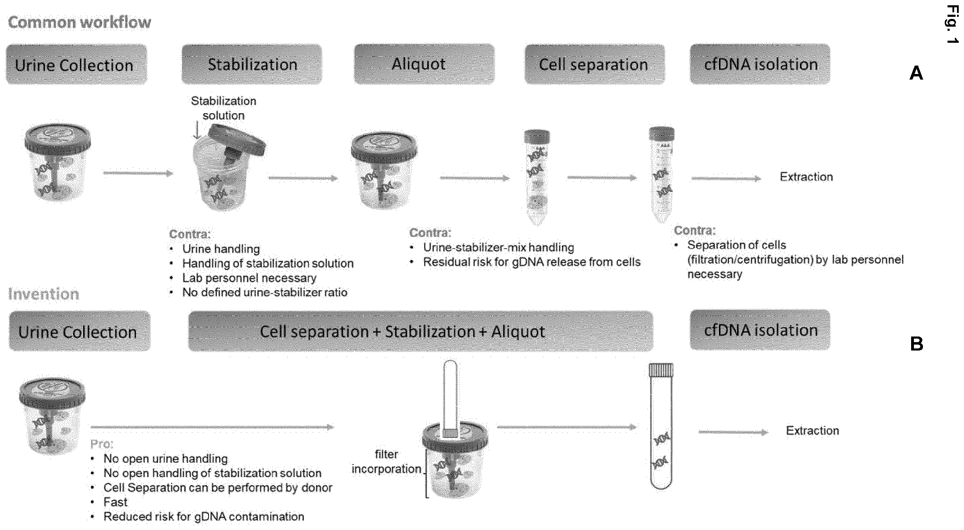

**Figure 1****:** Illustrates differences between the common workflow for cfDNA isolation from urine **(A)** and the improved workflow due to the present invention **(B).** The workflow **(B)** also illustrates a preferred embodiment of the method according to the first aspect. As is also apparent from the illustration in **(B),** the method according to the first aspect is particularly suitable for stabilizing extracellular cell-free nucleic acids, such as cfDNA, cfRNA and extracellular vesicles, in a cell-depleted biological sample obtained from a fluid cell-containing biological sample, such as urine. The method is particularly suitable for urine stabilization. As shown in **(B),** urine is collected in a urine collection device, here a urine collection cup. An evacuated reception device, e.g. an evacuated tube, containing the stabilizing composition is connected to the cup via a needle that is included in the lid of the cup in a recess. The cap of the tube is pierced by the needle and the vacuum achieves an automatic inflow of a defined volume of urine from the collection cup, through a straw, which is in contact with the collected urine (a transfer element - in the illustrated embodiment it forms part of the lid of the collection cup), into the reception device (tube) that comprises the stabilizing composition. The transfer of the urine is thus driven by the reception device, in the illustrated embodiment by the vacuum of the evacuated tube. The separation of cells and optionally further undesired components e.g. by filtration automatically occurs during the process of urine transfer from the collection device (cup) to the reception device (tube). For example, a filter element can be included in the transfer element (straw) of the container-lid that reaches to the bottom of the cup. When the evacuated tube is mounted to the lid (e.g. into a recess) and pierced by the needle, urine is automatically sucked through the straw by the vacuum and forced to pass the filter element during the transfer into the reception device. Consequently, cells and optionally further undesired components are retained by the filter medium while the cell-depleted flowthrough passes the filter and enters the evacuated tube where it is automatically contacted with the stabilizing solution. The transfer element (straw) and the needle form part of a transfer device that is in the shown embodiment integrated into the lid of the collection cup. Said transfer device incorporates the separation device, e.g. a filter, thereby allowing automatic cell separation during the fluid transfer. Accordingly, no separate or manual cell separation step is required, as a cell-depleted biological sample is automatically contacted with the stabilizing composition in the reception device. The methods of the present invention are also suitable for preparation of other analyte targets from liquid biopsies, such as pathogens including respiratory viruses, e.g. in mouthwashes, saliva and bronchial lavages, and their nucleic acids. Furthermore, the cells and optional further undesired components retained in the separation device can be analysed separately as is described in further detail herein.

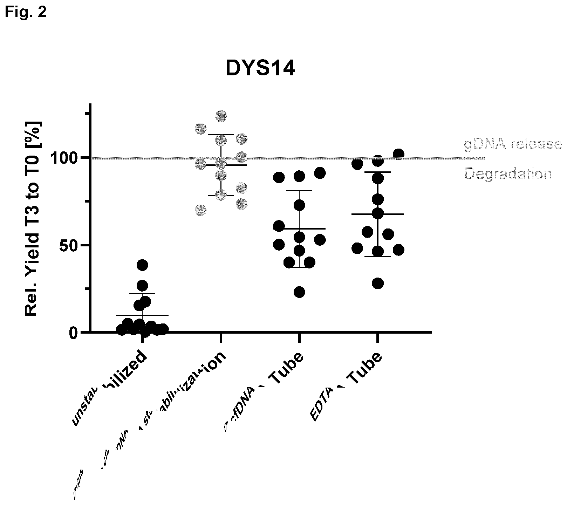

**Figure 2****:** Requirements for urine extracellular nucleic acid-specific preservation. Cell-free male urine was spiked into female urine. The mixture was either processed unstabilized or stabilized with either a urine extracellular DNA-specific preservative, which was prepared according to WO 2020/141187 A1, a blood ccfDNA-dedicated preservation tube (PAXgene^{®} Blood ccfDNA Kit, PreAnalytiX) or with an EDTA tube. Female cell separation by centrifugation followed by cfDNA isolation was performed directly after donation (T0) and three days after donation (T3). The level of the male-specific marker DYS14 was determined by qPCR. As no male cells were included in the processed urine, changes in DYS14 levels were caused only by degradation and not cellular DNA release. The relative yield between T0 and T3 indicated a strong degradation of cfDNA for unstabilized urine. ccfDNA and EDTA tubes showed reduced degradation. However, only the urine-dedicated preservative reached a relative yield of around 100% which indicates stability and a stop of degradation.

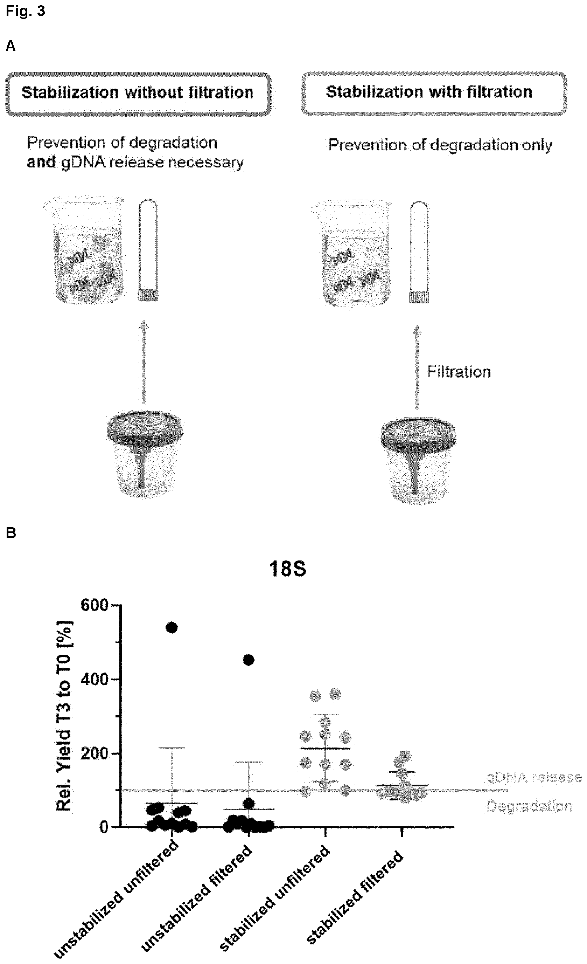

**Figure 3**: (A) To reliably detect the true levels of extracellular nucleic acids in urine at the timepoint of collection, the degradation of cell-free nucleic acids as well as the release of cellular nucleic acids from cells contained in the sample must be reduced or prevented. Effective stabilization of the extracellular nucleic acid population contained in the biological sample therefore requires the reduction or prevention of several processes, namely in particular (1) the degradation of extracellular nucleic acids and (2) the release of cellular nucleic acids such as gDNA from cells that are contained in the collected sample. The present invention advantageously separates the contained cells directly after sample collection during the transfer into the collection device, so that an already cell-depleted biological fluid is contacted with the stabilization composition that is comprised in the evacuated reception device. The requirement for the stabilization composition would mainly be the prevention of cfDNA degradation. **(B)** The non-crosslinking urine extracellular nucleic acid-specific stabilizing solution stopped degradation but did not completely reduce release of cellular DNA. The experiment was performed as described in Figure 2. 18S was analyzed as a marker for total DNA. An increase of 18S by around 2x (200%) over the time of three days indicated gDNA release. Filtration of urine before stabilization reduced the relative yield of 18S to about 100% demonstrating stability and a prevention of cellular DNA release.

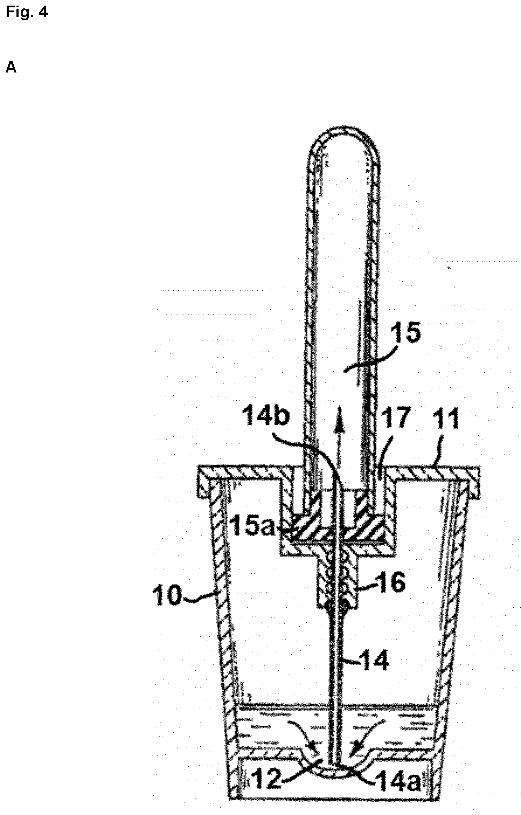

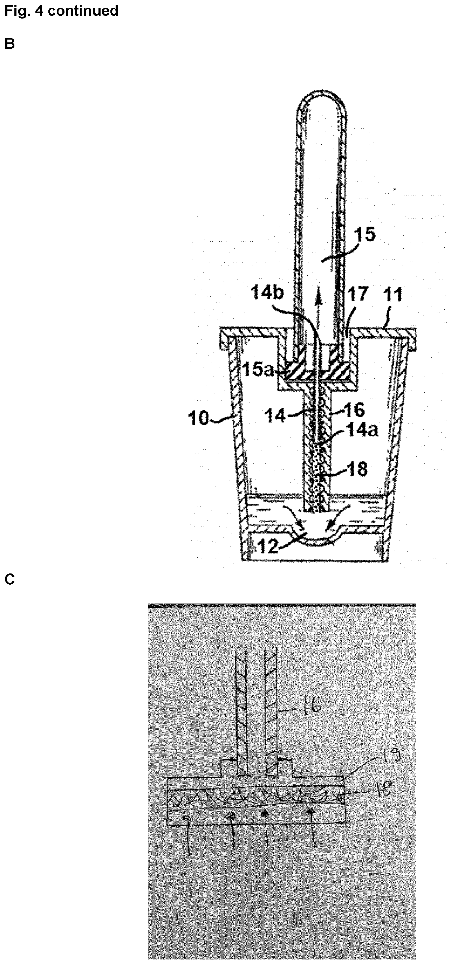

**Figure 4**: (A) Longitudinal section through the assembly according to one embodiment of the present invention. **(B)** Longitudinal section through the assembly according to a further embodiment of the present invention. **(C)** shows a separation device with a housing that comprises a separation element (filter) that can be removably assembled to the transfer element or connecting element of the transfer device.

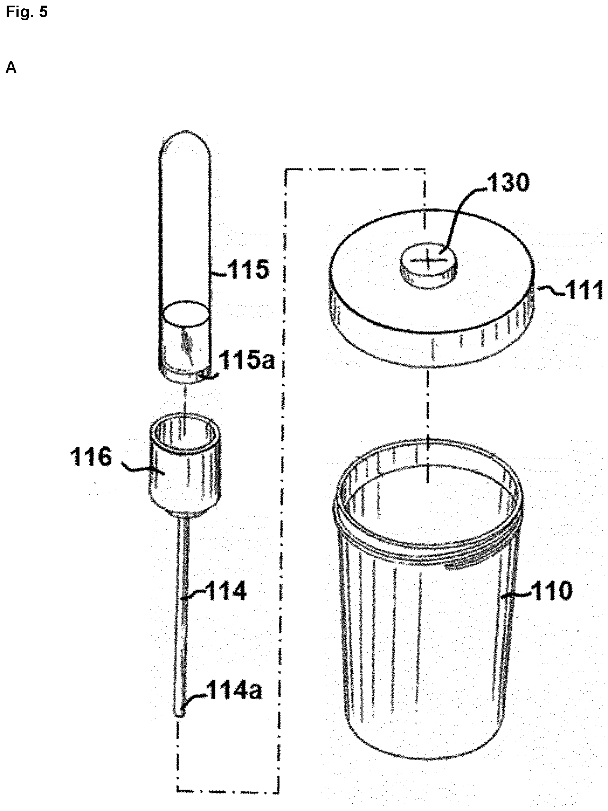

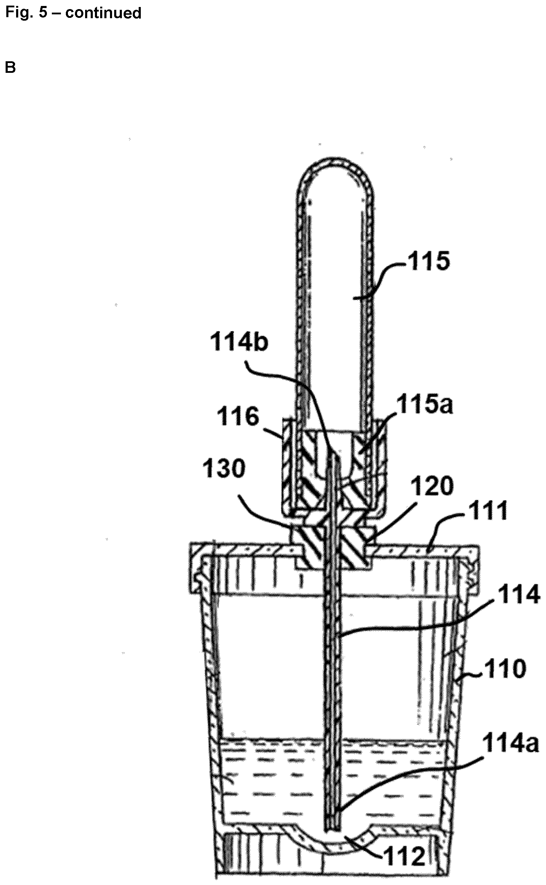

**Figure 5**: (A) Exploded perspective of the assembly according to an alternative embodiment of the present invention. **(B)** Longitudinal section through the assembly according to the exploded perspective of the assembly shown in **(A)** according to one embodiment of the present invention.

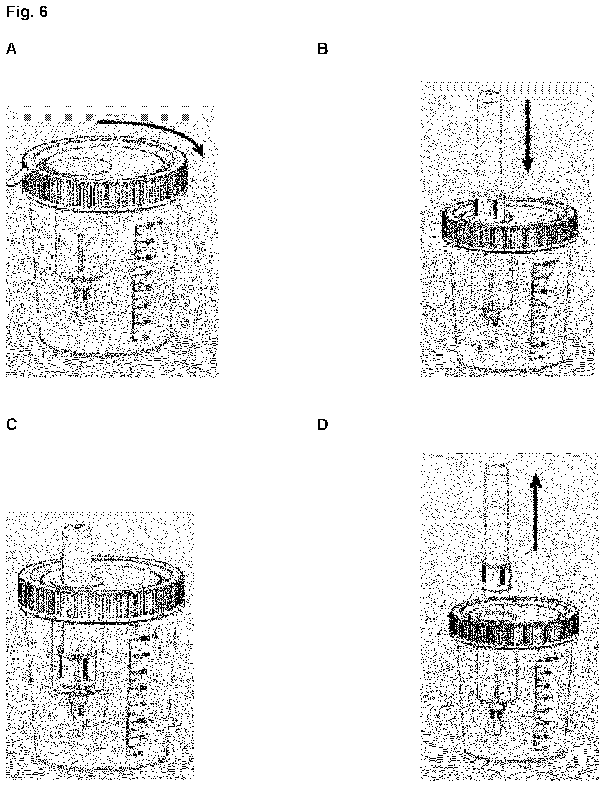

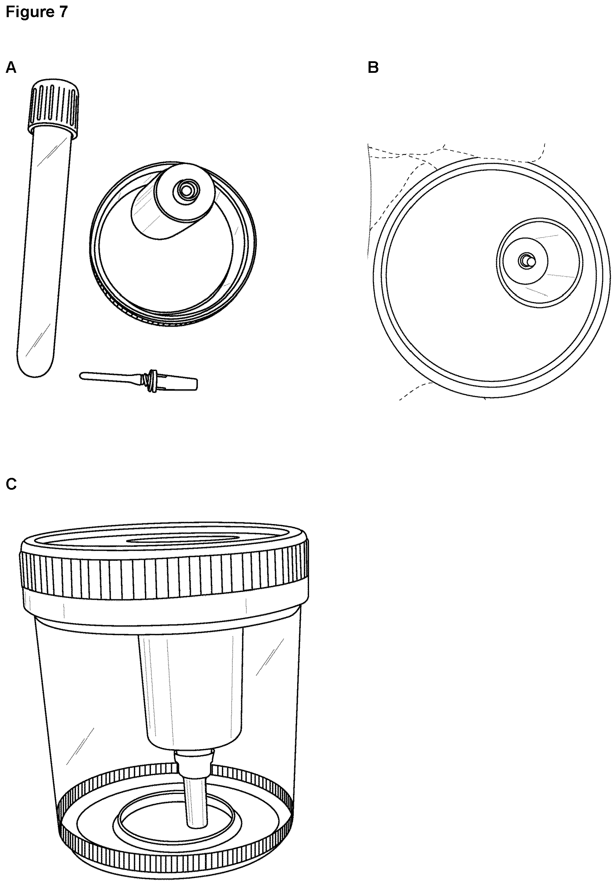

**Figures 6** and **7** illustrate further embodiments of the invention wherein urine collection systems are modified to incorporate a separation device that is capable of separating cells from the cell-containing urine sample so that a cell-depleted biological sample is contacted with a stabilizing composition that is comprised in the reception device.

### Reference signs

- 10/110:: collection device
- 11/111:: closing means
- 12/112:: reception (e.g. a rim or deepening) at the bottom of the collection device
- 14/114:: connecting element
- 14a/114a:: distal end of the connecting element
- 14b/114b:: proximal end of the connecting element comprising a tip
- 15/115:: reception tube
- 15a/115a:: lid of reception device
- 16/116:: transfer element
- 17/117:: recess
- 18:: separation device
- 19: removable separation device
- 120:: access port
- 130:: self-sealing cover means

### DETAILED DESCRIPTION OF THIS INVENTION

The different aspects of the present disclosure are now described in further detail:

### THE METHOD ACCORDING TO THE FIRST ASPECT

According to a first aspect, a method is provided for stabilizing extracellular nucleic acids contained in a fluid cell-depleted biological sample obtained from a fluid cell-containing biological sample, which preferably is a urine sample, said method comprising
(a) obtaining a fluid cell-containing biological sample in a collection device;
(b) establishing a connection to a reception device which comprises a stabilizing composition suitable for stabilizing extracellular nucleic acids;
(c) transferring the obtained cell-containing biological sample through a separation device, thereby separating cells and providing a cell-depleted biological sample and contacting the cell-depleted biological sample with the stabilizing composition comprised in the reception device, wherein preferably, the transfer is driven by the reception device.

In step (a), a fluid cell-containing biological sample, which preferably is a body fluid such as urine, is obtained in a collection device. The cell-containing biological sample may be a body fluid, preferably selected from urine, mouth washes, saliva, sputum, bronchial lavages, blood, plasma, serum, edema, pleural effusions, breast milk, tears, sweat, cerebrospinal fluid, synovial fluid, semen, vaginal fluid, ascitic fluid and amniotic fluid. The present invention is particularly suitable for processing body fluids hat can be collected in large volumes, such as urine. All embodiments described herein specifically apply to and are disclosed for the embodiment wherein the cell-containing biological sample is urine. The processing of urine and mouth wash is particularly advantageous with the present invention.

The fluid cell-containing biological sample may be directly collected from a donor into a collection device. For instance, the donor collects urine into a urine collection cup. The sample, such as urine, may also be transferred from another container (such as a catheter bag) into the collection device. Step (a) preferably comprises closing the collection device comprising the fluid cell-containing biological sample by a closing means, preferably a lid. The closing means may be removably attachable to the collection device, e.g. by screw fastening.

Suitable collection devices for collecting fluid cell-containing biological samples are known in the art and also commercially available. The collection device may be e.g. a urine cup or a pediatric bag. The collection device may be formed of a transparent plastic or any other suitable material. Suitable collection containers and cups, respectively, are well known in the art and are, for instance, described in DE3029631C2 and EP1284160B1. In case the cell-containing biological sample is urine, the collection device may be provided by a urine collection assembly, such as a urine collection cup and a lid. Accordingly, urine is collected in a urine collection cup, e.g. the second morning urine. Suitable collection devices are used in the examples and are also commercially available (e.g. BD Vacutainer^{®} urine collection cup).

In step (b), a connection to a reception device which comprises a stabilizing composition suitable for stabilizing extracellular nucleic acids is established. The connection between the collection device and the reception device may be established by a transfer device which in preferred embodiments comprises a connecting element, such as a needle or cannula, and a transfer element, such as a hollow transfer tube, as is described in further detail below.

### The separation device

In step (c), the cell-containing biological sample is transferred through a separation device. The separation device is capable of separating cells from the cell-containing biological sample whereby a cell-depleted biological sample is provided that enters the reception device and is contacted with the stabilizing composition that is comprised in the reception device. As is described herein, the transfer of the cell-containing biological sample through the separation device and of the cell-depleted biological sample into the reception device can advantageously be driven by the reception device. E.g. where the reception device is an evacuated reception device, the transfer can be advantageously driven by the vacuum of the reception device which aspires the cell-containing biological sample from the collection device into the reception device, whereby the cell-containing biological sample is forced through the separation device during said passage.

A core element of the present invention is the use of the separation device which retains and thus separates cells from the fluid cell-containing biological sample whereby a cell-depleted biological sample is provided that is contacted in the reception device with the stabilizing composition. The cell separation may advantageously occur in an automated manner during the transfer of the cell-containing biological sample and significantly improves the stabilization of extracellular nucleic acids because contamination and dilution of the extracellular nucleic acids by intracellular nucleic acids released from cells is reduced or even eliminated. As the cells are directly removed, this also reduces the risk that nucleases are released from cells. This overall improves the stabilization of the extracellular nucleic acids. As explained in further detail herein, the method also increases the safety since no open handling of the collected sample and the stabilizing solution is required. This also allows sterile handling of the sample during processing and prevents contamination by the environment. Moreover, no trained lab personnel is required for cell separation or stabilization of the sample.

The separation device comprises or consists of a separation element that is capable of retaining cells contained in the collected biological sample. The separation device may achieve cell separation by size selection and/or achieves or assists cell separation by the binding characteristics, i.e. the surface chemistry of the separation device, respectively the separation element. As is illustrated in the examples, the separation element may be provided by a filter or by beads. In preferred embodiments, the separation element is porous. The pore size can be chosen by the skilled person so that it allows the separation of the desired cells and optionally further components that shall be removed from the cell-containing sample. Considering common fluid body samples, such as urine, the pore size is chosen so that the separation element is capable of retaining mammalian cells, in particular human cells. In embodiments, the separation element is capable of separating cells and optionally at least one of debris, precipitates and pathogens. The separation element preferably allows passage of extracellular vesicles, so that the cell-depleted biological sample comprises extracellular vesicles, such as exosomes, including the extracellular nucleic acids that are comprised therein. The separation element may comprise functional groups, such as charged groups, to assist the separation of the cells and optionally further components.

The separation element is preferably a porous filter, optionally a membrane, sponge, mesh or sieve. As noted, the pore size can be chosen so that cells are retained by the separation element.

The material of the separation element may be selected from cellulose acetate/polycarbonate, track-etched polycarbonate membrane, nylon membrane, nitrocellulose membrane, antibody-coupled beads, silica membrane, filter sponge/foam filter, cotton wool, polyester fiber, polyester mesh, polyester wool, polypropylene, polybutylene terephthalate, negatively charged polyester, positively charged polyester, noncharged polyurethane, and leukoreduction filter. Suitable separation elements, in particular filters, can be chosen by the skilled person according to the specific requirements.

According to one embodiment, the separation device retains cells and optionally further components comprised in the fluid cell-containing biological sample and hinders said cells and optionally further components from passing through the separation element. According to one embodiment, the separation device, respectively the separation element, retains mammalian cells. In embodiments, the separation device also retains (i) debris, (ii) precipitates, and/or (iii) pathogens, such as bacteria, viruses and/or fungi that may be comprised in the collected cell-containing sample. In certain embodiments, pathogens, such as viruses and/or bacteria, e.g. respiratory viruses can pass through the separation device and remain present in the obtained cell-depleted biological sample. The separation device, respectively the separation element, can be selected according to the components that shall be retained or shall pass through.

According to one embodiment, the separation element retains the cells to be removed and optionally the further components due to size selection and/or the surface chemistry. According to one embodiment, the separation element is porous. The separation element has a pore size that enables the separation of mammalian cells, in particular human cells, that are comprised in the processed cell-containing biological sample. In embodiments, the pore size is chosen so that cells that are smaller than mammalian cells, such as pathogen cells, are separated in addition to mammalian cells. In embodiments, the pore size is chosen so that cell debris is separated in addition to the cells that are to be removed by the separation device. The pore size of the separation element may be in the range of 0.1 µm to 10 µm, 0.2 µm to 5 µm, 0.5 µm to 3 µm, 0.5 µm to 2 µm and 0.5 µm to 1 µm. In certain embodiments, the pore size is about 0.8 µm. As demonstrated in the examples a pore size of 0.8 µm is particularly advantageous to retain cells and debris present in urine and thereby significantly reducing the amount of contaminating nucleic acids. In case it is desired to separate only the mammalian cells but e.g. not debris, the pore size could be 5µm or more. E.g. normal or malignant urothelial cells comprised in urine typically have a diameter of >20 µm. In embodiments, pathogenic cells such as prokaryotes can also pass the separation element and are thus comprised in the cell-depleted fluid that is obtained after the cell-containing biological sample (e.g. urine or mouth wash) has passed the separation element. Separation elements with different pore sizes, such as various filters, are well-known to the skilled person and also commercially available.

In embodiments, the separation device may be removed for further processing of the retained material (e.g. the separated cells, cellular components, pathogens, debris, etc.). For instance, the retained material may be used as input for e.g. nucleic acid extraction, cultivation, cytology or further downstream applications. This allows analyzing the captured material (such as cells, cellular components) comprised in the fluid biological sample (e.g. urine). For instance, the separation device, respectively the retained cells may be transferred to a lysis solution for lysing the cells, cellular components and/or pathogens (e.g. viruses, bacteria, fungi). Subsequently, proteins and nucleic acids can be isolated and/or detected by any suitable methods known to the skilled person. Accordingly, more data can be obtained for diagnosing a medical condition or for assessing the overall status of the subject.

The separation device for retaining cells may be comprised in the collection device and/or in the reception device. It may furthermore be comprised or connected to a transfer device. Various options for placing the separation device, respectively the separation element, are described elsewhere herein.

### The reception device

The reception device comprises the stabilizing composition that is suitable for stabilizing extracellular nucleic acids in the cell-depleted biological sample. It can be made of glass, plastic or other suitable materials. The reception device may be made of a transparent material. The reception device may be closed by a closure. In embodiments, the reception device has an open top, a bottom, and a sidewall extending therebetween defining a chamber. The stabilizing composition may be comprised in the chamber. According to one embodiment the reception device is a tube, the bottom is a closed bottom, and the reception device further comprises a closure such as a cap at the open top. The reception device may be a tube having an open end sealed by a septum.

According to one embodiment, the reception device comprises a self-sealing closure (e.g. a lid, cap or elastic stopper) that is pierceable by the needle or cannula of a transfer device (described elsewhere herein). The closure is self-sealing so that no fluid can leak from the reception device. According to one embodiment, piercing of the closure of the reception device by an element of the transfer device establishes fluid communication between the collection device and the reception device.

In preferred embodiments, the reception device comprises a chamber that is at a reduced pressure to provide an evacuated reception device. The reduced pressure is preferably selected to draw a specified predefined volume of liquid sample into the reception device. In preferred embodiments, the reception device is thus an evacuated reception device, such as an evacuated tube. It is evacuated to an internal pressure below atmospheric pressure. In step (c), the transfer of the cell-containing biological sample from the collection device through the separation device can thus be driven by the vacuum of the reception device. As the reception device is air evacuated, a sample of the body fluid specimen, such as urine, is drawn through the transfer device into the reception device. This is advantageous as the transfer can be achieved in an automated manner upon connecting the evacuated reception device with the collection device. To establish this connection, common systems may be used, such as a transfer device as is described herein. After the desired amount of sample is collected within the reception device, the reception device may be removed. If desired, further reception devices may be connected to draw additional cell-depleted biological samples from the same collection device. This is particularly suitable for urine. Connecting two or more reception devices to the same collection device provides two or more stabilized cell-depleted biological samples for analysis, e.g. for different target analytes.

As noted, the evacuation is preferably effective to draw a specific volume of the biological sample into the interior of the reception device. This ensures that the desired amount of cell-depleted biological sample is contacted with the pre-filled amount of the stabilizing composition comprised in the reception device, and accordingly ensures that the stabilization is efficient. E.g. the reception device is pre-filled with a defined amount of the stabilizing composition and is provided with a defined vacuum and sealed with a septum. The septum is constructed such that it is compatible with the standard sampling accessories (e.g. needles, cannula, etc.). When contacted with the cell-depleted biological sample, preferably via a transfer device (described elsewhere herein), a specific amount of biological sample that is predetermined by the vacuum is collected into the reception device. According to one embodiment, the reception device is designed for receiving a volume in the range of 5 ml to 30 ml, preferably 7 ml to 25 ml, such as 10 ml to 25 ml.

Evacuated tubes containing a stabilization composition for extracellular nucleic acids are known to the skilled person (e.g. for blood: WO2013/045457, WO2013/045458, WO2014/146780, WO2014/146782, WO2017/085321; for urine: WO2020/141187A1) and are also commercially available (e.g. BD Vacutainer^{®} Urinalysis Preservative Tube).

According to one embodiment, the reception device comprises the separation device, preferably wherein the separation device is a filter. For instance, the separation device may be positioned behind the tip of the connecting element when the self-sealing closure of the reception device is pierced by the tip of the connecting element. This ensures that the cell-containing biological sample that enters the reception device through the tip of the connecting element must pass through the separation device before it reaches the interior or chamber of the reception device that comprises the stabilizing composition. During passage of the fluid cell-containing biological sample from the outlet of the connecting element into the interior/chamber of the reception device that comprises the stabilizing composition, the cells are retained by the separation device while the cell-depleted fluid can pass through the separation device into the interior of the reception device, where it comes into contact with the stabilizing composition. Once the transfer is terminated, the reception device may be positioned such that the closure is positioned upwards, thereby preventing contact between the stabilized biological sample and the separation device comprising the retained cells. The separation device may also be assembled in a removable manner, so that it can be removed from the reception device after the cell-depleted fluid has entered the interior/chamber of the reception device.

According to another embodiment, the reception device is provided by a syringe device and wherein in (c) the transfer of the cell-containing biological sample through the separation device is achieved by drawing up the syringe. The volume drawn can thus be determined by the user who controls the plunger of the syringe. This embodiment also allows the use of a needle-free transfer device. E.g. the syringe comprising the stabilizing composition may be inserted into the recess of the closure means, e.g. the lid, of the collection device. The tip of the syringe may reach into a transfer device, such as a hollow tube, that is attached to the bottom of the recess. The passage may be closed by a self-sealing septum that can be penetrated by the tip of the syringe. Upon drawing up the plunger, the cell-containing biological sample is aspired from the collection device and transferred into the syringe. During this transfer, the cell-containing biological sample passes through a separation device whereby cells are separated so that the cell-depleted biological sample is contacted with the stabilizing composition comprised in the collection chamber of the syringe. As described herein, the separation device, such as a porous filter, may be comprised in or attached to e.g. the transfer device, the collection device or the reception device.

### The transfer device

For establishing the connection in step (b), preferably a transfer device is used that establishes or contributes to establish a connection between the collection device and the reception device. The connection enables the transfer of fluid from the collection device into the reception device. Corresponding transfer devices are known in the art and e.g. used in commercially available systems, such as urine collection systems.

According to one embodiment, the separation device is comprised in or attached to the transfer device. The fluid cell-containing biological sample passes through the established connection and the separation device. During the transfer, cells and optionally further components contained in the fluid cell-containing biological sample are retained by the separation device while the fluid passes the separation device, thereby providing a cell-depleted biological sample.

The transfer device may be provided by a single element or may be assembled from at least two elements. The transfer device or element thereof may be mounted to or formed integrally with the collection device or the closure means for the collection device. In common embodiments, the transfer device or element thereof is mounted to or formed integrally with the closure means for the collection device.

The transfer device may comprise or consist of a connecting element, wherein the connecting element has a distal end directed into the collection device and a proximal end directed to the exterior of the collection device, wherein the proximal end preferably comprises a tip. The connecting element may be provided by a needle or a cannula. The tip of the connecting element, e.g. a needle or cannula, is preferably accessible from the outer side of the closed collection device such that the reception device, which preferably is an evacuated tube, can be connected to the connecting element. As is illustrated in the examples, the tip may extend into a recess in the closure means of the collection device, wherein the recess is designed to receive at least the top of the reception device. E.g. where the reception device comprises a piercable self-sealing lid, the tip of the connecting element may pierce the lid, thereby providing a fluid connection between the collection device and the reception device and allowing the fluid cell-containing biological sample to pass from the collection device into the reception device, wherein during said passage, the cell-containing biological sample passes the separation device for cell removal. The tip of the connecting element may be covered by a removable cover means to increase the safety and sterility during handling and/or during storage of the fluid cell-containing biological sample in the collection device. The cover means may be a seal extending over a recess in the closure means of the collective device. This seal may be removed prior to connection of the reception device. The cover means may also be provided by a protective sleeve around the tip of the needle or of the cannula. According to one embodiment, the protective sleeve may be removed or pressed down to uncover the tip of the needle or of the cannula.

According to one embodiment, the connecting element comprises the separation device. The separation device may also be removably attached to the connecting element, e.g. to the distal end of the connecting element that reaches into the collection device. According to one embodiment, the separation device can thus be removed from the connecting element.

The transfer device may also comprise or consist of a transfer element that receives the connecting element. The transfer element is preferably a hollow tube-like element that extends into the collection device. In embodiments, the transfer element is provided by a straw that extends from the closure means, e.g. the bottom of a recess, into the collection device when it is closed. According to an alternative embodiment, the transfer element comprises a cup-like receptable having a size that is adapted to receiving the reception device (see also Figure 5). The transfer element may comprise the separation device. The separation device may also be removably attached, e.g. screwed or clamped, to the transfer element, e.g. to the distal end of the transfer element that reaches into the collection device. According to one embodiment, the separation device can thus be removed from the transfer element.

In preferred embodiments, the transfer device comprises the transfer element and the connecting element. The transfer element may position the connecting element so that it can be connected to the reception device. The connecting element may extend at least partially through the transfer element. The transfer element extends into the collection device. The transfer element may e.g. be provided by a straw that extends from the closure means into the collection device. In embodiments, the distal end of the connecting element extending into the collection device is shorter than the transfer element. When the fluid cell-containing biological sample is contained in the collection device, the distal end of the straw is covered by the cell-containing biological sample. When the reception device is connected to the proximal end of the connecting element, the cell-containing biological sample is drawn up through the straw and further passes the connecting element into the interior of the reception device. In other embodiments, the connecting element is longer than the transfer element so that the distal end of the connecting element reaching into the collection device is covered by the cell-containing biological sample. When a reception device is connected to the proximal end of the connecting element, the cell-containing biological sample is drawn up through the connecting element into the interior of the reception device.

The transfer device may also be provided by a cup-like receptable having a size that is adapted to receiving the reception device and an elongated hollow tube extending into the collection device. According to this embodiment, the distal end of the elongated hollow tube of the transfer element extending into the collection device nearly reaches the bottom of the collection device and is covered by the obtained cell-containing biological sample. The distal end of the connecting element extending into the collection device is shorter than the elongated hollow tube of the transfer element and thus is located within the interior of the elongated hollow tube. When the evacuated reception device according to the present invention is connected to the proximal end of the connecting element, the cell-containing biological sample is drawn up through the elongated hollow tube of the transfer element and then through the connecting element into the interior of the reception device.

The connecting element may removably attached or securely attached to the transfer element. In embodiments, the connecting element is comprised in a coupling element that can be removably attached, e.g. screwed or clipped, to the transfer element (see also Figures 6 and 7). According to one embodiment, the coupling element comprises a separation device, optionally wherein the separation device comprises or consists of a filter. The coupling element may be easily removed, e.g. by unscrewing or unclipping, and the retained material such as cells may be further analysed or nucleic acids extracted therefrom.

As noted, the separation device for separating cells in step (c) may be comprised in at least one element of the transfer device, optionally wherein the separation device comprises or consists of a filter. Furthermore, at least two elements of the transfer device may comprise a separation device, optionally wherein each separation device is provided by a filter, optionally wherein the pore size of the filters comprised in the different elements differ from each other. According to one embodiment, the transfer element and/or the connecting element comprises a separation device, optionally wherein the separation device comprises or consists of a filter.

According to one embodiment, the transfer element comprises the separation device, optionally wherein the separation device is provided by a filter. According to an alternative embodiment, the connecting element comprises the separation device, optionally wherein the separation device is provided by a filter. According to a further embodiment, both the transfer tube and the connecting element each comprise a separation device. In the latter embodiment, each separation device may be provided by a filter. According to one embodiment, the pore size of the filter comprised in the connecting element differs from the pore size of the filter comprised in the transfer element. This may be particularly advantageous in case cells and/or components of different size present in the fluid cell-containing biological sample shall be retained separately according to their size. Thus, the components retained in the separation device can be extracted and analysed. For instance, the separation device which is first passed by the cell-containing biological sample may have larger pores and retains larger cells while smaller components and particles, such as pathogens or subcellular components, may be retained in the second separation device that has smaller pores. In case the transfer element extending into the collection device is longer than the connecting element, the separation device with the larger pore size may be comprised in the transfer element and the separation device with the smaller pore size is comprised in the connecting element. Accordingly, larger cells and/or components present in the fluid cell-containing biological sample are retained in the separation device comprised in the transfer tube while smaller cells, pathogens, particles and/or subcellular components are retained in the separation device comprised in the connecting element. According to one embodiment, separation is provided by size selection and surface chemistry, e.g. in case the transfer element comprises a filter having a specifically defined pore size while the connecting element comprises beads or *vice versa.* Thus, the separation devices comprised in the transfer element and in the connecting element may differ in their type. Different combinations of separation devices in the transfer element and in the connecting element are covered by the disclosure of the present invention and the skilled person can select suitable combinations with regard to the respective requirements.

The transfer device or at least an element thereof, such as the transfer element, is connected to the closing means or the collection device. According to one embodiment, the transfer device has one or more of the following characteristics:
(i) it is attached to the wall of the collection device and extends through the wall of the collection device, optionally wherein the transfer device is removable from the wall of the collection device; or
(ii) it is attached to the bottom of the collection device and extends through the bottom of the collection device, optionally wherein the transfer device is removable from the bottom of the collection device.

According to one embodiment, the transfer device is attached to the wall of the collection device such that the end of the transfer device that extends (optionally horizontal) into the collection device is covered by the fluid cell-containing biological sample when the fluid cell-containing biological sample has been obtained in the collection device. According to an alternative embodiment, the transfer device is attached to the bottom of the collection device such that the end of the transfer device that extends (optionally vertical) into the collection device is covered by the fluid cell-containing biological sample when the fluid cell-containing biological sample has been obtained in the collection device. The wall or bottom of the collection device may comprise a recess of a size adapted to receive the reception device, wherein the proximal end of the connecting element comprising the tip extends into the recess, such that the tip is positioned on the outer side of the wall or of the bottom of the collection device, and wherein the distal end of the connecting element extends into the interior of the collection device, preferably wherein the connecting element is provided by a needle or a cannula, wherein the transfer tube is attached to the bottom of the recess on the inner side of the wall or of the bottom of the collection container and extends into the interior of the collection device.

In preferred embodiments, the transfer device or element thereof is connected to the closing means of the collection device which is preferably provided by a lid. The transfer device or at least an element thereof may be formed integrally (i.e. from one piece) to the closing means or the collection device, preferably the closing means. The transfer device may also be removably attachable to the closing means or the collection device, preferably the closing means which is provided by a lid.

According to one embodiment, the transfer device is integrated in the closing means, wherein the closing means is a lid, wherein the lid has an upper side and a lower side and comprises a recess of a size adapted to receive the reception device, wherein the connecting element is mounted to the bottom of the recess, wherein the proximal end of the connecting element comprising the tip is positioned within the recess, such that the proximal end of the connecting element comprising the tip is positioned on the upper side of the lid, and wherein the distal end of the connecting element extends into the collection device, preferably wherein the connecting element is provided by a needle or a cannula, wherein the transfer element is attached to the bottom of the recess on the lower side of the lid and extends into the collection device. According to one embodiment, the transfer element forms the extension of the recess. According to a preferred embodiment, a cross section of the transfer tube is smaller than the cross section of the recess.

According to one embodiment, the transfer device or an element thereof is attached permanently to the closing means of the collection device. The transfer device may be formed integrally in the closing means of the collection device (integrated transfer device). Integrated transfer devices - without a separation device - are, for instance, described in DE3029631C2. This embodiment is advantageous as no separate transfer device is required for transferring the cell-containing biological sample from the collection device to the reception device. The transfer of the biological sample is thus simple, fast and convenient. Furthermore, no open handling for transferring the biological sample is required. According to one embodiment, the transfer element is provided by a straw extending from the closing means (e.g. a lid) into the collection device. According to one embodiment, the distal end of the transfer element nearly reaches the bottom of the collection device. According to an alternative embodiment, the distal end of the connecting element extends through the distal end of the transfer element and thus the distal end of the transfer element that extends into the collection device is shorter than the distal end of the connecting element that extends into the collection device. According to this embodiment, the distal end of the connecting element nearly reaches the bottom of the collection device. According to a further embodiment the distal end of the transfer element and the distal end of the connecting element extending into the collection device are of the same length and both nearly reach the bottom of the collection device.

In advantageous embodiments, the closing means comprises a recess adapted to at least partially receive the reception device. The recess may be provided by an inner protrusion that extends into the collection device when the closing means is mounted to the collection device. A transfer device comprising a connecting element may be connected to the closing means, e.g. formed integrally or assembled thereto, wherein the tip of the connecting element extends into the recess. The transfer element may be connected to the bottom of the recess, e.g. formed integrally. Hence, the transfer device may comprise a transfer element and a connecting element and is connected to the closing means, wherein the closing means is a lid, wherein the lid has an upper side and a lower side and comprises a recess adapted to receive the reception device, wherein the transfer element of the transfer device is attached to the bottom of the recess on the lower side of the lid and extends into the collection device when the lid is closed, and wherein the connecting element is mounted to the bottom of the recess, preferably via the transfer element, wherein the proximal end of the connecting element comprising the tip is positioned within and extends into the recess, preferably wherein the connecting element is provided by a needle or a cannula. The connecting element may be comprised in a coupling element that is attached, e.g. screwed or clipped, to the transfer element.

The bottom of the collection device may have a reception, which can be provided e.g. as deepening or heightened rim, and into which the distal end of the transfer element and/or the distal end of the connecting element extends. This is particularly advantageous to reduce volume losses during sample processing.

In embodiments, the transfer device or at least an element thereof is removably attachable to the collection device or the closing means of the collection device. The transfer device may be removably attachable to the closing means of the collection device and wherein the transfer device comprises a transfer element and a connecting element, wherein at least an element of the transfer device is inserted into the collection device through an access port, wherein the access port is positioned in the closing means. The transfer element of the transfer device provides a recess, preferably a cup-like receptable, adapted to receive the reception device, wherein the cup-like receptable is positioned on the outer side of the access port. The proximal end of the connecting element comprising the tip is positioned within the cup-like receptable. The access port may be closed by a cover means, preferably wherein the cover means is an elastomeric self-sealing material. Removable transfer devices are exemplary illustrated in Example 4 and Figures 4 and 5 and are, for example, described in EP1284160B1. According to one embodiment, the distal end of the connecting element or the transfer element is inserted into the collection device through an access port, wherein the access port is positioned in the closing means, in the wall of the collection device or in the bottom of the collection device, respectively. According to one embodiment, the access port is covered by a cover means which may be made from an elastomeric self-sealing material. The removable transfer device may comprise a transfer element with a cup-like receptable of a size adapted to receive the reception device. The cup-like receptable is positioned on the outer side of the access port when the transfer device is inserted into the collection device. According to one embodiment, the proximal end of the connecting element is positioned within the cup-like receptable, so that the tip pierces the closure of the reception device when it is inserted into the receptacle thereby allowing fluid communication between the collection device and the reception device.

### The stabilizing composition comprised in the reception device

The reception device comprises a stabilizing composition which is contacted with the cell-depleted biological sample for stabilization of the contained extracellular nucleic acids. The stabilizing composition comprised in the reception device protects extracellular nucleic acids comprised in the cell-depleted biological sample from degradation.

The cell-depleted biological sample that contacts the stabilizing composition in the reception device comprises extracellular nucleic acids, such as cell-free DNA (cfDNA) and/or cell-free RNA (cfRNA), and optionally extracellular vesicles, such as exosomes. It may also comprise viral nucleic acids and/or pathogens, such as viruses and/or bacteria, such as respiratory viruses. The separation device does not retain the desired target analytes so that they remain in the cell-depleted biological sample after passage through the separation device and are comprised in the reception device. The methods of the present invention are particularly suitable to obtain a cell-depleted biological sample comprising extracellular nucleic acids.

The term "extracellular nucleic acids" or "extracellular nucleic acid" as used herein, in particular refers to nucleic acids that are not contained in cells. Respective extracellular nucleic acids are also often referred to as cell-free nucleic acids. These terms are used as synonyms herein. Hence, extracellular nucleic acids usually are present exterior of a cell or exterior of a plurality of cells within a sample. The term "extracellular nucleic acids" refers to extracellular DNA and extracellular RNA. Cell-free DNA in urine is of specific interest. Examples of typical extracellular nucleic acids that are found in the cell-free fraction (respectively portion) of fluid cell-containing biological samples, in particular body fluids such as urine, include but are not limited to mammalian extracellular nucleic acids such as e.g. extracellular tumor-associated or tumor-derived DNA and/or RNA, other extracellular disease-related DNA and/or RNA, epigenetically modified DNA, fetal DNA. Extracellular nucleic acids may also include non-mammalian extracellular nucleic acids such as e.g. viral nucleic acids or other pathogen nucleic acids that are released into the extracellular nucleic acid population e.g. from prokaryotes (e.g. bacteria), viruses, eukaryotic parasites or fungi. The extracellular nucleic acid population may comprise some intracellular nucleic acids that were released from damaged or dying cells either prior or during collection of the biological sample or during cell separation by the separation device.

The term extracellular nucleic acids in particular refers to mammalian extracellular nucleic acids, such as mammalian extracellular DNA and mammalian extracellular RNA. Examples include but are not limited to disease-associated or disease-derived extracellular nucleic acids such e.g. as tumor-associated or tumor-derived extracellular nucleic acids, extracellular nucleic acids released due to inflammations or injuries, in particular traumata, extracellular nucleic acids related to and/or released due to other diseases, extracellular nucleic acids derived from organ transplants, or extracellular nucleic acids derived from a fetus. Extracellular nucleic acids, such as extracellular RNA, may also be comprised in exosomes. Mammalian extracellular DNA is of specific interest as is also shown by the examples.

The term "extracellular nucleic acid population" or "extracellular DNA population" and "extracellular RNA population", respectively, as used herein in particular refers to the collective of different extracellular nucleic acids, such as DNA and RNA, that are comprised in the biological sample. Of specific interest is the extracellular DNA population in a urine sample. A urine sample usually comprises a characteristic and thus unique extracellular nucleic acid population. Thus, the type, kind, ratio and/or the amount of one or more extracellular nucleic acids comprised in the extracellular nucleic acid population of a urine sample may be important sample characteristics. It is therefore advantageous to stabilize and thus substantially preserve said extracellular DNA population at the state wherein the urine sample is collected, as the comprised type, composition and/or the amount of one or more extracellular nucleic acids comprised in the extracellular nucleic acid population of a sample can provide valuable medical, prognostic or diagnostic information. Therefore, it is advantageous if the profile of the extracellular nucleic acid population, e.g. of the cell-free DNA, is efficiently stabilized over the intended stabilization period. The stabilization described herein reduces the degradation of the comprised cell-free DNA. Thus, a substantial preservation of the extracellular DNA population is achieved.

A further issue after collecting the cell-containing biological sample (e.g. a urine sample) is the contamination and dilution of the cell-free nucleic by intracellular nucleic acids, in particular fragmented genomic DNA, that originates from damaged or dying cells that are contained in the sample. Due to the early cell separation that is achieved with the present invention, contaminations and hence a dilution of the extracellular nucleic acids by intracellular nucleic acids, in particular by genomic DNA, is reduced. It is advantageous to remove cells and optionally further undesired components from the cell-containing biological sample (e.g. a urine sample) and efficiently inhibit degradation of the comprised cell-free nucleic acids in the cell-depleted biological sample.

According to one embodiment, the cell separation and stabilization method of the present invention reduces the increase of DNA that results from a release of genomic DNA from cells contained in the biological sample during the stabilization period compared to an unstabilized unfiltered sample and also in comparison to a corresponding stabilized unfiltered sample. This is in particular achieved by the separation of cells and optionally further cellular components, which is optionally further supported by the stabilization of cells and cellular components that may remain in the cell-depleted biological sample (such stabilization reduces or prevents the release of further genomic DNA from remaining cells). The release of DNA can be determined e.g. by quantifying the ribosomal 18S DNA as is described herein in the example section. As is shown in the examples, the filtration and stabilization achievable with the teachings of the present invention may reduce this release of DNA over the stabilization period.

As is demonstrated in the examples, the filtration and stabilization method according to the present invention effectively reduces the dilution of the target analyte by undesired components, such as intracellular nucleic acids, and efficiently stabilizes extracellular (cell-free) nucleic acids, which is particularly valuable for diagnostic purposes. The filtration and stabilization technology according to the present invention is of particular advantage in this respect because it substantially preserves the extracellular nucleic acids comprised in the cell-depleted biological sample and e.g. inhibits degradation of the comprised extracellular nucleic acids (preferably at least by 50%, at least by 60% or at least by 70% over the stabilization period compared to timepoint 0, directly after stabilization). An effective stabilization of cell-free nucleic acids, such as cell-free DNA, in the cell-depleted biological sample after cell separation is achievable with the method of the present invention for a period of at least three days, at least 5 days and also longer. However, the samples may also be further processed earlier, if desired. It is not necessary to make use of the full achievable stabilization period.

To efficiently reduce the degradation of the extracellular nucleic acids in the cell-depleted biological sample, the stabilizing composition comprises a nuclease inhibitor. The nuclease inhibitor is preferably a chelating agent, more preferably ethylenediamine tetraacetic acid (EDTA). A chelating agent is an organic compound that is capable of forming coordinate bonds with metals through two or more atoms of the organic compound. Chelating agents include, but are not limited to ethylenedinitrilotetraacetic acid (EDTA), diethylenetriaminepentaacetic acid (DTPA), ethylene glycol tetraacetic acid (EGTA) and N,N-bis(carboxymethyl)glycine (NTA) and furthermore salts of carboxylic acids such as citrate or oxalate and any combination thereof.

EDTA is the preferred chelating agent, as it effectively inhibits the degradation of extracellular nucleic acids. All disclosures and embodiments described in this application for a chelating agent in general, specifically apply and particularly refer to the preferred embodiment EDTA even if not explicitly stated. As used herein, the term "EDTA" indicates *inter alia* the EDTA portion of an EDTA compound such as, for example, K₂EDTA, K₃EDTA or Na₂EDTA. Using a chelating agent such as EDTA has the advantageous effect that nucleases such as DNases and RNases are inhibited, thereby reducing degradation of the cell-free nucleic acids comprised in the cell-depleted biological sample (in particular a cell-depleted urine sample) by nucleases. Therefore, the use of a chelating agent such as EDTA for stabilizing the cell-depleted biological sample is highly advantageous.

According to one embodiment, the final concentration of the chelating agent, preferably EDTA, in the stabilized cell-depleted biological sample and thus in the mixture that is obtained when contacting the cell-depleted biological sample with the stabilizing composition comprised in the reception device lies in a range of 0.9mg/ml to 40mg/ml, such as e.g. 2mg/ml to 40mg/ml. Exemplary and preferred ranges are disclosed in the following, wherein the disclosed ranges are particularly suitable for the use of EDTA as chelating agent. The final concentration in the stabilized cell-depleted biological sample may be at least 4mg/ml, at least 5 mg/ml, at least 7mg/ml or at least 10 mg/ml. In embodiments, the final concentration in the stabilized cell-depleted biological sample is at least 12 mg/ml. The use of a chelating agent, such as preferably EDTA, in higher concentrations for stabilizing the cell-depleted biological sample, e.g. obtained from urine, is advantageous to achieve a strong stabilizing effect. Therefore, in embodiments, the final concentration of the chelating agent, which preferably is EDTA, in the stabilized cell-depleted biological sample is at least 7mg/ml, preferably at least 10mg/ml. According to one embodiment, the final concentration of the chelating agent, preferably EDTA, lies in a range of 10mg/ml to 35 mg/ml or 10mg/ml to 30mg/ml. Also suitable is a final concentration in a range of 12mg/ml to 30mg/ml or 15mg/ml to 25mg/ml.

According to one embodiment, the final concentration of the chelating agent, preferably EDTA, in the stabilized cell-depleted biological sample lies in a range of 2mM to 100mM, preferably 10mM to 50mM or 25 to 50mM. In the examples, EDTA was used in a final concentration of approx. 38mM in the stabilized cell-depleted biological sample.

In preferred embodiments, the stabilizing composition is a liquid composition. The chelating agent, preferably EDTA, may be comprised in the stabilising composition in a concentration up to the solubility limit. According to one embodiment, a liquid composition is provided as stabilizing composition which comprises a chelating agent, preferably EDTA such as K₂EDTA, in a final concentration of at least 15mM, such as e.g. at least 25mM, at least 50mM, at least 75mM, at least 100mM, at least 200mM or at least 250mM. A high concentration of the chelating agent in the stabilizing composition is advantageous. According to one embodiment, the stabilizing composition comprises the chelating agent, preferably EDTA, in a concentration of 100mM up to the solubility limit or 150mM up to the solubility limit. Suitable final concentration ranges in the stabilizing composition include 100mM to 500mM, 125mM to 450mM and 150mM to 400mM. Also higher concentrations may be used that lie in a range of 175mM to 400mM, 200mM to 400mM or 250mM to 350mM in the stabilizing composition. These concentrations are particularly suitable for EDTA as chelating agent. In embodiments, only a chelating agent is used for stabilization of the extracellular nucleic acids.

The stabilizing composition may also comprise an additive for cell and/or cellular component stabilization. Including such additive may assist the stabilization of any remaining cells or cellular components (that were not removed by the separation device) in the cell-depleted biological sample, thereby reducing the risk of releasing intracellular nucleic acids. The stabilizing composition may comprise at least one volume excluding polymer. Examples of suitable volume excluding polymers include, but are not limited to, polyethylene glycol (PEG), glycerol, trehalose, dextrans and derivatives such as dextran sulfate and dextran acetate, and hydrophilic polymers such as polyvinyl alcohol, polyvinyl acetate and polyvinyl sulfate. In embodiments, the stabilizing composition does not comprise a volume excluding polymer. Where the stabilizing composition comprises a volume excluding polymer, it is preferably a poly(oxyethylene) polymer, more preferably polyethylene glycol. The polyethylene glycol may be unbranched and may be unsubstituted or substituted. Known substituted forms of polyethylene glycol include alkylpolyethylene glycols that are e.g. substituted at one or both ends with a C1-C5 alkyl group. Most preferably, unsubstituted polyethylene glycol of the formula HO-(CH₂CH₂O)ₙ-H is used. All disclosures and embodiments described in this application for the poly(oxyethylene) polymer in general, specifically apply and particularly refer to the preferred embodiment polyethylene glycol and specifically unsubstituted polyethylene glycol even if not explicitly stated. The poly(oxyethylene) polymer can be used in various molecular weights, e.g. in the range of 200 to 35000. Higher molecular weight poly(oxyethylene) polymers having a molecular weight of at least 1500 were found to be more effective stabilizing agents than lower molecular weight poly(oxyethylene) polymers. Lower molecular weight poly(oxyethylene) polymers must be used in higher concentration. For some applications it is preferred though to keep the amount or concentration of additives used for stabilization low. Therefore, in embodiments, a high molecular weight poly(oxyethylene) polymer is comprised in the stabilizing composition for stabilization, as it allows to use lower concentrations of the poly(oxyethylene) polymer. The high molecular weight poly(oxyethylene) polymer may have a molecular weight that lies in a range selected from 1500 to 40000. Suitable ranges include 2000 to 35000, 2500 to 30000, 3000 to 25000, 3500 to 20000 and 4000 to 15000. Preferred is a range of 5000 to 20000, such as 5000 to 15000. PEG 10000 was also used in the examples. The high molecular weight poly(oxyethylene) polymer is used in a concentration, wherein it supports the stabilization of the cell-depleted biological sample. Suitable concentrations can be determined by the skilled person, for example by testing different concentrations of a specific high molecular weight poly(oxyethylene) polymer in the test assays described in the examples. The achieved stabilization effect and the preferred concentration may depend on whether one or more additional stabilizing agents are used. Preferred combinations are described herein. According to one embodiment, the mixture that is obtained after contacting the cell-depleted biological sample with the stabilizing solution comprising the high molecular weight poly(oxyethylene) polymer and optionally further additives comprises the high molecular weight poly(oxyethylene) polymer in a final concentration that lies in a range of 0.05% to 8% (w/v). Exemplary suitable ranges for the final concentration in the stabilized cell-depleted biological sample include 0.1% to 4% (w/v), 0.2% to 3% (w/v), 0.25% to 2.5% (w/v) and 0.3% to 2% (w/v). Using a high molecular weight poly(oxyethylene) polymer in a concentration of 1.25% (w/v) or less or 1% (w/v) or less and in particular in a concentration of 0.75% (w/v) or less can be advantageous in certain embodiments. The downstream processing, in particular the purification of cell-free nucleic acids from the stabilized sample, may be improved. It is advantageous to use a stabilization technology that is compatible with most standard nucleic acid isolation methods. According to one embodiment, the poly(oxyethylene) polymer, which preferably is a polyethylene glycol, used for stabilization has a molecular weight below 1500 and may be a low molecular weight poly(oxyethylene) polymer having a molecular weight of 1000 or less. It is used in a concentration, wherein it can support the cell-depleted biological sample stabilizing effect. Suitable concentrations for different sample types can be determined by the skilled person. A respective poly(oxyethylene) polymer, such as a low molecular weight poly(oxyethylene) polymer having a molecular weight of 1000 or less, can be present in the mixture that is obtained after contacting the cell-depleted biological sample with the stabilizing composition comprising said poly(oxyethylene) polymer and optionally further additives in a final concentration that lies in a range of 0.5% to 10%. Exemplary ranges include 1.5% to 9%, 2% to 8%, 2 to 7%, 2.5% to 6% and preferably 2.5% to 5%. The percentage values refer to (w/v) in case the poly(oxyethylene) polymer is a solid and to (v/v) in case the poly(oxyethylene) polymer is a liquid. The low molecular weight poly(oxyethylene) polymer may have a molecular weight that lies in a range selected from 100 to 1000, 150 to 800 and 150 to 700. The molecular weight may lie in the range of 200 to 600 and more preferably 200 to 500 such as 200 to 400. PEG 300 was also used in the examples.

In embodiments, the stabilizing composition comprises at least two poly(oxyethylene) polymers, which differ in their molecular weight. They may be of the same kind and preferably both are a polyethylene glycol such as preferably an unsubstituted polyethylene glycol. According to one embodiment, the difference in the molecular weight is at least 500, at least 750, or at least 1000. According to one embodiment, the difference in the molecular weight is at least 2500, at least 3500 or at least 5000. It is advantageous to use two poly(oxyethylene) polymers that differ in their molecular weight. The stabilization effect of poly(oxyethylene) polymers appears to depend on their molecular weight. It was found that the higher molecular weight polymers provide a better stabilization efficiency. This embodiment wherein at least two poly(oxyethylene) polymers are used for stabilization that differ in their molecular weight is advantageous, because it allows to provide balanced compositions of poly(oxyethylene) polymers having the desired characteristics with respect to the stabilization effect to be achieved and characteristics required e.g. for certain downstream uses, such as the purification of cell-free DNA from the cell-depleted stabilized urine sample. It is also referred to WO 2020/141187 which describes such stabilizing compositions that can be used as stabilizing compositions for the present invention. According to one embodiment, a high molecular weight poly(oxyethylene) polymer as defined above which has a molecular weight of at least 1500, preferably in a range of 5000 to 20000 such as 5000 to 15000, is used in combination with a low molecular weight poly(oxyethylene) polymer having a molecular weight of 1000 or less, preferably in a range of 200 to 600, and the urine sample is contacted with both types of poly(oxyethylene) polymers. Suitable embodiments are described herein. Using a high molecular weight poly(oxyethylene) polymer in combination with a low molecular weight poly(oxyethylene) polymer is advantageous, because the low molecular weight poly(oxyethylene) polymer allows to reduce the concentration of the high molecular weight poly(oxyethylene) polymer required to achieve an effective stabilization of the cell-depleted biological sample. This embodiment is advantageous because it provides more freedom with respect to the volume or amount of stabilizing composition that can be used. Suitable and preferred concentrations and concentration ranges for the high and low molecular weight poly(oxyethylene) polymer are described above and may also be used in the embodiment wherein both types of polymers are used in combination.

In embodiments, the stabilizing composition comprises a preservative for inhibiting bacterial growth. The method may thus achieve that bacterial growth is inhibited in the stabilized cell-depleted biological sample, such as a stabilized cell-depleted urine sample. Bacterial load in urine samples can vary between a high load to almost sterile. Furthermore, although the urine sample is filtered through the separation device, thereby also optionally separating bacteria from the flowthrough in case a suitable separation device is selected, some bacteria may remain in the cell-depleted urine sample. Bacteria rapidly grow in urine. It is therefore advantageous to inhibit their growth efficiently because this increases the stabilization effect and the quality of the recovered nucleic acids. Bacteria internalize and digest nucleic acids. Bacteria are a source for nucleic acid degrading enzymes such as DNases and also RNases. Therefore, inhibiting bacterial growth in the stabilized urine sample supports the prevention of nucleic acid degradation by such mechanisms. It furthermore reduces the risk that bacterial DNA is copurified from the sample and might interfere in downstream assays, such as sequencing applications or PCR. Chelating agents such as EDTA can achieve in higher concentrations a bacteriostatic effect. To achieve inhibition of bacterial growth in the stabilized urine sample, the chelating agent, which is preferably EDTA, may thus be used in a high concentration. Suitable high concentrations were described above. Alternatively, or additionally, the stabilizing composition may comprise a preservative for inhibiting bacterial growth. If such preservative is used in addition to the chelating agent, which preferably is EDTA, the preservative accordingly differs from the used chelating agent. According to one embodiment, the stabilizing composition thus comprises an additional preservative for inhibiting bacterial growth. The preservative may be an antimicrobial and preferably has antibacterial and optionally also antifungal effects. The preservative may comprise or consist of a chemical compound. The preservative may also comprise or consist of two or more compounds that inhibit bacterial growth. The preservative is used in a concentration so that bacterial growth is inhibited in the stabilized cell-depleted biological sample. Suitable concentrations can be determined by the skilled person for individual preservatives. The final concentration of the preservative in the stabilized cell-depleted biological sample may lie e.g. in the range of 0.01 to 0.7% w/v, such as 0.03 to 0.5 % w/v, or may lie in the range of 0.01 to 0.7% v/v, such as 0.03 to 0.5 %v/v. According to one embodiment, the preservative may comprise or consist of sodium azide. It may be used in a final concentration of e.g. 0.01% to 0.5% (w/v) in the stabilized cell-depleted biological sample. According to embodiments, the final concentration of sodium azide in the stabilized cell-depleted biological sample is in the range of 0.02% to 0.4% (w/v) such as 0.03% to 0.35% (w/v) or 0.04% to 0.3% (w/v). Furthermore, the preservative may comprise or consist of at least one isothiazolinone compound, preferably methylchloroisothiazolinone and/or methylisothiazolinone. These compounds may be mixed in a ratio of 3:1. A commercially available preservative for inhibiting bacterial growth that comprises isothiazoline compounds is ProClin300. As is demonstrated by the examples, it is suitable for use in the stabilization of urine. It may be used in a final concentration of e.g. 0.03% to 0.1% (v/v) such as 0.05% (v/v) in the stabilized cell-depleted biological sample. Further examples of preservatives that can be used include but are not limited to chlorhexidine or sodium borate. Further preservatives for inhibiting bacterial growth and useful concentrations can be determined by the skilled person following the disclosure of the present application. According to one embodiment, the preservative is comprised in a concentration in the stabilizing composition so that bacterial growth is inhibited in the stabilized cell-depleted biological sample. Suitable concentrations can be determined by the skilled person for individual preservatives. The final concentration in the stabilizing composition may lie e.g. in the range of 0.1 to 5.5% w/v, e.g. 0.2 to 3.75 % w/v or may lie in the range of 0.1 to 5.5% v/v, e.g. 0.2 to 3.75 % v/v. According to one embodiment, the preservative may comprise or consist of sodium azide. The stabilization composition may comprise sodium azide in a final concentration of e.g. 0.15% to 3.75% (w/v) or 0.2% to 3.5% (w/v). According to embodiments, the final concentration in the stabilization composition is 0.3% to 3% (w/v) or 0.3% to 2.5% (w/v). Furthermore, the preservative may comprise or consist of at least one isothiazolinone compound, preferably methylchloroisothiazolinone and/or methylisothiazolinone. These compounds may be mixed in a ratio of 3:1. A commercially available preservative for inhibiting bacterial growth that comprises isothiazoline compounds is ProClin300. As is demonstrated by the examples, it is suitable for use in the stabilization of urine. It may be comprised in the stabilization composition in a final concentration of e.g. 0.2% to 1% (v/v) or 0.3 to 0.5% (v/v) such as e.g. 0.34% (v/v).

In embodiments, the stabilizing composition may comprise at least one caspase inhibitor. It is possible to generate reversible or irreversible inhibitors of caspase activation by coupling caspase-specific peptides to e.g. aldehyde, nitrile or ketone compounds. E.g. fluoromethyl ketone (FMK) derivatized peptides such as Z-VAD-FMK act as effective irreversible inhibitors with no added cytotoxic effects. Inhibitors synthesized with a benzyloxycarbonyl group (BOC) at the N-terminus and O-methyl side chains exhibit enhanced cellular permability. Further suitable caspase inhibitors are synthesized with a phenoxy group at the C-terminus. An example is Q-VD-OPh which is a cell permeable, irreversible broad-spectrum caspase inhibitor that is even more effective in preventing apoptosis and thus supporting the stabilization than the caspase inhibitor Z-VAD-FMK. According to one embodiment, the caspase inhibitor is a pancaspase inhibitor and thus is a broad spectrum caspase inhibitor. According to one embodiment, the caspase inhibitor is a caspase-specific peptide. Preferably, said caspase-specific peptide is modified. It may be modified by an aldehyde, nitrile or ketone compound. According to one embodiment, the caspase specific peptide is modified, preferably at the carboxyl terminus, with an O-Phenoxy (OPh) or a fluoromethyl ketone (FMK) group. According to one embodiment, the caspase inhibitor is selected from the group consisting of Q-VD-OPh and Z-VAD(OMe)-FMK. In a preferred embodiment, Q-VD-OPh, which is a broad spectrum inhibitor for caspases, is used for stabilization. Q-VD-OPh is cell permeable and inhibits cell death by apoptosis. Q-VD-OPh is not toxic to cells even at extremely high concentrations and comprises a carboxy terminal phenoxy group conjugated to the amino acids valine and aspartate. It is equally effective in preventing apoptosis mediated by the three major apoptotic pathways, caspase-9 and caspase-3, caspase-8 and caspase-10, and caspase-12 (Caserta et al, 2003). The stabilized cell-depleted biological sample, i.e. the mixture that is obtained after contacting the cell-depleted biological sample with the stabilizing composition comprising the caspase inhibitor and the further additives in the evacuated reception device may comprise the caspase inhibitor (or combination of caspase inhibitors) in a final concentration of at least 0.5µM. Embodiments include a concentration of at least 1µM, e.g. at least 1.5µM, at least 2µM or at least 2.5µM. Suitable final concentration ranges for the caspase inhibitor(s) when mixed with the cell-depleted biological sample and the further additives include but are not limited 1µM to 25µM. Embodiments include 1.5µM to 20µM and 2µM to 15µM. Preferred are final concentration ranges of 2µM to 12.5µM, 2.5µM to 10µM and 3µM to 7.5µM. The above-mentioned concentrations apply to the use of a single caspase inhibitor as well as to the use of a combination of caspase inhibitors. The aforementioned concentrations are in particular suitable when using a pancaspase inhibitor, in particular a modified caspase specific peptide such as Q-VD-OPh and/or Z-VAD(OMe)-FMK. Suitable concentration ranges for individual caspase inhibitors and/or for other urine samples can be determined by the skilled person. In the examples, a final caspase inhibitor (Q-VD-Oph) concentration of 5µM in the stabilized cell-depleted biological sample was used. According to one embodiment, the stabilization composition comprises a caspase inhibitor in a concentration from 1µM to 220 µM, e.g. 5 µM to 200 µM, 10 µM to 150 µM, 20.0 µM to 100 µM, and 25 µM to 50 µM. The stabilizing composition is preferably a liquid composition that can be filled e.g. in a reception device and is e.g. suitable for stabilizing a sample unit.

According to one embodiment, the stabilizing composition comprises one or more primary, secondary or tertiary amide as further stabilizing agent. Also combinations of two or more primary, secondary or tertiary amides can be used. As can be seen from WO 2014/146781, primary and secondary carboxylic acid amides are capable of stabilizing the extracellular nucleic acid population over a broad concentration range. According to one embodiment, the primary carboxylic acid amide is selected from the group consisting of formamide, acetamide, propanamide and butanamide. According to one embodiment, the primary carboxylic acid is butanamide. The stabilization effect of butanamide on the extracellular nucleic acid population is also described in detail in WO 2014/146780 and WO 2014/146782. The amide preferably is a carboxylic acid amide. Suitable concentrations for different primary, secondary or tertiary amides can be determined by the skilled person, e.g. by testing different concentrations in the test assays described in the examples. Generally, the mixture that is obtained when contacting the urine sample with the one or more primary, secondary or tertiary amides and the further additives may comprise said amide (or combination of amides) in a final concentration of at least 0.05%. E.g. the final concentration may be at least 0.1 %, at least 0.25%, at least 0.5% or at least 0.75%. Suitable concentration ranges include but are not limited to 0.1% to 10%, 0.25% to 7.5%, 0.3% to 5%, 0.4% to 3%, 0.5% to 2%, 0.6% to 1.8% or 0.75% to 1.5%. Concentrations or concentration ranges indicated in percentage values as used herein are in particular given as percentage weight per volume (w/v) for solid amides and as percentage volume per volume (v/v) for liquid amides. According to one embodiment, the primary, secondary or tertiary amide is a compound according to formula 1 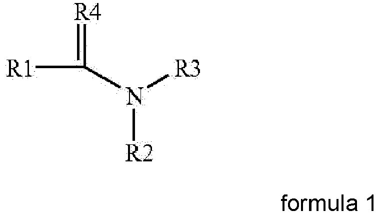 wherein R1 is a hydrogen residue or an alkyl residue, preferably a C1-C5 alkyl residue, a C1-C4 alkyl residue or a C1-C3 alkyl residue, more preferred a C1-C2 alkyl residue, R2 and R3 are identical or different and are selected from a hydrogen residue and a hydrocarbon residue, preferably an alkyl residue, with a length of the carbon chain of 1 - 20 atoms arranged in a linear or branched manner, and R4 is an oxygen, sulphur or selenium residue, preferably R4 is oxygen. Also a combination of one or more compounds according to formula 1 can be used for stabilization. In embodiments, wherein R1 is an alkyl residue, a chain length of 1 or 2 is preferred for R1. R2 and/or R3 of the compound according to formula 1 are identical or different and are selected from a hydrogen residue and a hydrocarbon residue, which preferably is an alkyl residue. According to one embodiment, R2 and R3 are both hydrogen. According to one embodiment, one of R2 and R3 is a hydrogen and the other is a hydrocarbon residue. According to one embodiment, R2 and R3 are identical or different hydrocarbon residues. The hydrocarbon residues R2 and/or R3 can be selected independently of one another from the group comprising alkyl, including short chain alkyl and long-chain alkyl, alkenyl, alkoxy, long-chain alkoxy, cycloalkyl, aryl, haloalkyl, alkylsilyl, alkylsilyloxy, alkylene, alkenediyl, arylene, carboxylates and carbonyl. The chain length n of R2 and/or R3 can in particular have the values 1, 2, 3, 4, 5, 6, 7, 8, 9, 10, 11, 12, 13, 14, 15, 16, 17, 18, 19 and 20. According to one embodiment, R2 and R3 have a length of the carbon chain of 1-10, preferably 1 to 5, more preferred 1 to 2. According to one embodiment, R2 and/or R3 are alkyl residues, preferably C1-C5 alkyl residues. Preferably, the compound according to formula 1 is a carboxylic acid amide so that R4 is oxygen. It can be a primary, secondary or tertiary carboxylic acid amide. According to one embodiment, the compound according to formula 1 is a N,N-dialkyl-carboxylic acid amide. Preferred R1, R2, R3 and R4 groups are described above. According to one embodiment, the compound according to formula 1 is selected from the group consisting of N,N-dimethylacetamide, N,N-diethylacetamide, N,N-dimethylformamide and N,N-diethylformamide. Also suitable are the respective thio analogues, which comprise sulphur instead of oxygen as R4. According to one embodiment, the stabilizing composition comprises a N,N-dialkylpropanamide, such as N, N-dimethylpropanamide.

Additional additives can also be comprised in the stabilizing composition to further support the stabilization of the cell-depleted biological sample, respectively support the preservation of the comprised extracellular nucleic acid population. When choosing a respective further additive for the stabilizing composition to support stabilization, care should be taken not to compromise and/or counteract the stabilizing effect of the core stabilizing agents. Thus, no additives should be used in concentrations that lead to a degradation of nucleic acids contained in the cell-depleted biological sample, such as in particular cell-free DNA and/or cell-free RNA. The extracellular nucleic acid population preferably substantially corresponds to or at least closely resembles the extracellular nucleic acid population present at the time of collection of the fluid cell-containing biological sample (e.g. urine), filtration and stabilization. Furthermore, the used stabilizing composition preferably does not comprise any additives that induce a modification of the extracellular nucleic acids in the cell-depleted biological samples, such as crosslinking agents. Therefore, it preferably does not comprise formaldehyde, paraformaldehyde or a formaldehyde releaser. Advantageously, nucleic acids can be efficiently isolated from respectively stabilized cell-depleted biological samples using different standard methods as no crosslinking of the sample occurs due to the stabilization. This greatly simplifies and improves the standardization of molecular analysis that relies on the analysis of nucleic acids obtained from the stabilized cell-depleted biological sample.

Suitable stabilizing compositions that can be used for stabilizing urine are disclosed in WO 2020/141187 A1, herein incorporated by reference.

As disclosed herein, the stabilizing composition comprised in the reception device may be a liquid or a dried composition. The stabilizing composition comprised in the reception device may be provided in a dry form, a semi-liquid form or as liquid. The advantage of using a solid stabilizing composition is that solids are often chemically more stable. According to one embodiment, the inner wall of the reception device is treated/covered with a stabilizing composition. Said composition can be applied to the inner walls using e.g. a spray-dry-method. Liquid removal techniques can be performed on the stabilizing composition in order to obtain a substantially solid-state protective composition. Liquid removal conditions may be such that they result in removal of sufficient liquid so that the resulting composition is in the form of a film, gel or other substantially solid or highly viscous layer. For example, it may result in a substantially immobile coating (preferably a coating that can be re-dissolved or otherwise dispersed upon contact with the cell-depleted biological sample, such as a cell-depleted urine sample). It is possible that lyophilization or other techniques may be employed for realizing a substantially solid form of the stabilizing composition (e.g., in the form of one or more pellet). Thus, liquid removal conditions may be such that they result in a material that upon contact with the cell-depleted biological sample the stabilizing composition will disperse in the sample, and substantially preserve components (e.g., extracellular nucleic acids) in the sample.

Liquid compositions have the advantage that the mixture with the sample to be stabilized can be quickly achieved, thereby basically providing an immediate stabilizing effect as soon as the cell-depleted biological sample comes into contact with the liquid stabilizing composition. According to a preferred embodiment, the reception device comprises a liquid stabilizing composition. Furthermore, liquid compositions are advantageous if larger amounts of stabilizing compositions are used which accordingly, cannot or are difficult to spray-dry or if the composition hampers providing a dry composition. The use of a liquid composition also allows to adjust or control the pH of the cell-depleted biological sample.

To provide a liquid stabilizing solution, the components of the stabilizing composition can be comprised, respectively can be dissolved in a solvent, e.g. water, a buffer, e.g. a biological buffer such as TRIS, MOPS, TES, HEPES, TAPSO, Tricine, Bicine, TAPS, borate buffer, CHES buffer or buffers on the basis of sodium or potassium phosphate such as PBS. A further buffering agent comprises carbonate. Particularly preferred is the use of a Tris buffer. The pH of the biological sample, such as e.g. urine, may vary from acidic to basic from donor to donor and additionally depending on the donor's conditions. It is thus advantageous to comprise the buffering agent in a sufficient concentration to achieve an efficient pH adjustment and stabilization despite potential variations in the pH of the collected biological sample, such as the collected urine sample, to be stabilized. The buffering agent is also advantageous to ensure a certain pH of the stabilization solution over the shelf life to thereby maintain the pH of the stabilization solution so that it is optimal for stabilization of the sample. Suitable concentrations for the buffering agent can be determined by the skilled person. Furthermore, the components may be dissolved in, or the stabilizing composition may comprise a polar aprotic solvent such as dimethyl sulfoxide (DMSO). E.g. a liquid stabilisation composition of 0.5ml to 5ml, 1 ml to 4.5ml, preferably 2.5ml to 4ml such as 3ml to 3.8ml can be used. Such stabilization composition comprising the stabilizing agents, in particular in the concentrations indicated below, can be used for stabilizing e.g. 20ml to 25ml urine as also shown in the examples.

According to one embodiment, the stabilizing composition is a liquid composition that has a pH that lies in the range of 4 to 9.5, such as 4.3 to 9.5, 4.5 to 9.5, 4.7 to 9.5, 5 to 9.5, 5.5 to 9.5, 6 to 9.5, 6.5 to 9.5, 6.7 to 9, 7 to 9 or 7.2 to 9, preferably 7.2 to 7.8, such as 7.5. The stabilizing composition may be used to adjust the pH in the stabilized cell-depleted biological sample (e.g. urine sample). It can thereby be achieved that the pH of the stabilized cell-depleted biological sample lies in a certain pH range. For this purpose, the stabilizing composition that is contacted for stabilization with the cell-depleted biological sample preferably comprises a buffering agent. A stabilizing composition having a higher pH value, such as preferably 7 or higher such as 7.5, is more efficient in preserving the cell-free DNA, because degradation of cell-free DNA is more efficiently prevented compared to a stabilizing composition having an acidic pH. This is highly advantageous when intending to analyze cell-free DNA comprised in the urine sample.

According to one embodiment, the final pH of the stabilized cell-depleted biological sample, preferably a cell-depleted urine sample, lies in a range of 5.5 to 9.5, such as 6.0 to 9.0 or 6.0 to 8.0. The final pH of the stabilized cell-depleted biological sample may be ≤9, such as ≤8.75 or ≤8.5, or ≤8.0. In embodiments, the final pH of the stabilized cell-depleted biological sample, such as a stabilized cell-depleted urine sample, is in the range of pH 5.5 to 7.5, such as 6.0 to 7.0.

According to one embodiment, a liquid stabilizing composition is contacted with the cell-depleted biological sample in a volumetric ratio selected from 1:3 to 1:10, such as 1:5 to 1:10, 1:5 to 1:8, in particular about 1:6 or about 1:7. It is a particular advantage of the stabilizing composition of the present invention that stabilization of a large sample volume can be achieved with a small volume of the stabilizing composition. As is demonstrated in the examples, a volumetric ratio selected from about 1:5 or about 1:7 is particularly suitable.

### Preserving composition in the collection device

The collection device may also comprise a composition to assist the stabilization. In one embodiment, the collection device comprises a nuclease inhibitor, preferably a chelating agent such as EDTA, and/or a cell preserving composition. The nuclease inhibitor may be comprised in the cell preserving composition. The cell preserving composition in the collection device stabilizes cells and reduces cell lysis. It thereby reduces of prevents the release of cell contents, including intracellular nucleic acids, such as gDNA, and nucleases. The cell preservative may protect the cells comprised in the cell-containing biological sample from shear forces thereby reducing damage when the biological-sample passes through the separation device. This provides the advantage that an initial stabilization effect is already achieved upon first collection of the fluid cell-containing biological sample.

The composition comprised in the collection device may be a liquid or dry composition, preferably a spray-dried composition.

According to one embodiment, the collection device used for obtaining a fluid cell-containing biological sample in step a) comprises a chelating agent, such as preferably EDTA. The chelating agent may be incorporated in spray-dried form. Incorporating a chelating agent such as EDTA into the collection device that is used for initial collection of the fluid cell-containing biological sample (e.g. a urine cup), provides the further advantage that the final concentration of the chelating agent in the stabilized biological sample can be increased and/or the concentration of the chelating agent in the stabilization composition comprised in the reception device can be lowered. The chelating agent is preferably comprised in a cell preserving composition.

In embodiments, the collection device comprises a cell preserving composition that comprises a volume excluding polymer and/or an osmotic agent. The volume excluding polymer may be selected from a poly(oxyethylene) polymer, preferably polyethylene glycol (PEG), glycerol, trehalose, dextrans and derivatives thereof, optionally selected from dextran sulfate and dextran acetate, and hydrophilic polymers optionally selected from polyvinyl alcohol, polyvinyl acetate and polyvinyl sulfate. The osmotic agent may be selected from sugars, such as glucose or sucrose, sugar alcohols and salts such as NaCl and KCI. The volume excluding polymer and the osmotic agent act to stabilize cells and reduce cell lysis, thereby preventing the release of cell contents, including gDNA and nucleases. The osmotic agent creates a hypertonic solution, which causes water to be removed from the cells that are present in the sample, causing the cells to shrink which renders them more stable. When additionally incorporating a nuclease inhibitor, such as EDTA, nucleases will be inactivated, thereby preventing or reducing degradation of the cell-free nucleic acids.

The cell preserving composition may also comprise a cell-protective surfactant, such as poloxamer 188. It may furthermore comprise a metabolic inhibitor to reduce cell metabolism.

In embodiments, the composition comprised in the collection device is the same as the stabilizing composition in the reception device.

### Further steps

The stabilized cell-depleted biological sample can be stored and/or transported in the reception device prior to further processing. Since the majority of cells and optionally further undesired components are removed by passage through the separation device, the risk of further contamination and dilution of the extracellular nucleic acids during storage of the cell-depleted biological sample is reduced. In addition, the stabilizing composition prevents degradation of the extracellular nucleic acids and optionally stabilizes the remaining cells and/or undesired components. As demonstrated in the examples, using the methods of the present invention allows for stabilizing the cell-depleted biological sample without refrigeration or freezing for a prolonged time period. Thus, the samples can be kept at room temperature or even at elevated temperatures e.g. up to 30°C or even up to 40°C. According to one embodiment, a stabilization effect is achieved for at least three days, preferably at least four days, more preferred at least 6 days, at least 9 days or at least 10 days.

It is also within the scope of the present invention to include further steps to work up the stabilized cell-depleted biological sample and/or the cellular sample retained by the separation device.

Preferably, the method comprises purifying extracellular nucleic acids, such as preferably extracellular DNA and/or extracellular RNA, from the cell-depleted biological sample. According to one embodiment, nucleic acid isolation is performed within one day up to 3 days or within 2 days up to 9 days after the cell-containing biological sample was collected, filtered and stabilized according to the method according to the present invention.

The method may also comprise purifying intracellular nucleic acids, such as genomic DNA, from the cells that were retained by the separation device. The present method allows the separate processing and analysis of extracellular and intracellular nucleic acids, e.g. extracellular DNA and genomic DNA, comprised in the biological sample.

For isolating nucleic acids, any suitable nucleic acid isolation method can be used and suitable methods are well-known and commercially available. Examples for respective purification methods include but are not limited to extraction, solid-phase extraction, silica-based purification methods, magnetic particle-based purification, phenol-chloroform extraction, chromatography, anion-exchange chromatography (using anion-exchange surfaces), electrophoresis, filtration, precipitation and combinations thereof. According to one embodiment, nucleic acids are isolated from the cell-depleted biological sample using a chaotropic agent and/or alcohol. Preferably, the nucleic acids are isolated by binding them to a solid phase, preferably a solid phase comprising silica or carrying anion exchange functional groups. Respective methods are well-known in the prior art and thus, do not need any detailed description. Suitable methods and kits for isolating extracellular nucleic acids are also commercially available such as the QIAsymphony PAXgene@ Blood ccfDNA Kit (QIAGEN), QIAamp^{®} Circulating Nucleic Acid Kit (QIAGEN), QIAsymphony DSP circulating DNA Kit (QIAGEN), the Chemagic Circulating NA Kit (Chemagen), the NucleoSpin Plasma XS Kit (Macherey-Nagel), the Plasma/Serum Circulating DNA Purification Kit (Norgen Biotek), the Plasma/Serum Circulating RNA Purification Kit (Norgen Biotek), the High Pure Viral Nucleic Acid Large Volume Kit (Roche) and other commercially available kits suitable for extracting and purifying extracellular nucleic acids. According to one embodiment, nucleic acids are isolated by binding them to a solid phase comprising anion exchange groups. Suitable anion exchange groups are for example provided by amine groups. Binding preferably occurs at a pH below 7. Such anion exchange based nucleic acid isolation methods are known to the skilled person. According to one embodiment, the nucleic acids are extracellular nucleic acids. Suitable anion exchange-based methods are e.g. described in WO 2013/045432, herein incorporated by reference.

According to one embodiment, total nucleic acids are isolated from the cell-depleted biological sample. It is also within the scope of the present invention to isolate at least predominantly a specific target nucleic acid. A target nucleic acid can be e.g. a certain type of extracellular nucleic acid, e.g. DNA or RNA (includes miRNA, snRNA, lincRNA), preferably cell-free DNA. E.g. the target extracellular nucleic acid can be DNA and the non-target extracellular nucleic acid can be RNA. Target specific nucleic acid isolation methods which specifically aim at isolating DNA or RNA are also well known in the prior art and thus, do not need any detailed description herein. It is also within the scope of the present invention to specifically isolate specific target extracellular nucleic acids, e.g. by using appropriate probes coupled to a solid support that enable a sequence specific binding. The term target nucleic acid may also refer to nucleic acids having a certain length. Size selective nucleic acid, in particular DNA isolation methods, are known in the art. A classic method for isolating DNA of a target size involves the separation of the DNA in a gel, cutting out the desired gel band(s) and then isolating the DNA of the target size from the gel fragment(s). Another widely used technology is the size selective precipitation with polyethylene glycol based buffers (Lis and Schleif Nucleic Acids Res. 1975 Mar;2(3):383-9) or the binding/precipitation on carboxyl-functionalized beads (DeAngelis et al, Nuc. Acid. Res. 1995, Vol 23(22), 4742-3; US 5,898,071 und US 5,705,628, commercialized by Beckman-Coulter (AmPure XP; SPRIselect) and US 6,534,262). Furthermore, size selective isolation methods that are based on the use of solid supports comprising anion exchange groups and varying pH values are known. A size selective isolation provides further opportunities in order to reduce the amount of intracellular nucleic acids in the isolated extracellular nucleic acids.

According to one embodiment, the isolated nucleic acids are analysed and/or further processed, e.g. using a suitable assay and/or analytical methods. E.g. they can be identified, modified, contacted with at least one enzyme, amplified, reverse transcribed, cloned, sequenced, contacted with a probe, be detected (their presence or absence) and/or can be quantified. Respective methods are well-known in the prior art and are commonly applied in the medical, diagnostic and/or prognostic field in order to analyse extracellular nucleic acids such as cell-free DNA. Thus, after the extracellular nucleic acid of interest was isolated from the cell-depleted biological sample, it can be analysed e.g. to identify the presence, absence or severity of a disease state including but not being limited to a multitude of neoplastic diseases, in particular premalignancies and malignancies such as different forms of tumors or cancers. E.g. the isolated extracellular nucleic acids can be analysed in order to detect diagnostic and/or prognostic markers (e.g., fetal- or tumor-derived extracellular nucleic acids) in many fields of application, including but not limited to non-invasive prenatal genetic testing respectively screening, disease screening, pathogen screening, oncology, cancer screening, early stage cancer screening, cancer therapy monitoring, genetic testing (genotyping), infectious disease testing, injury diagnostics, trauma diagnostics, transplantation medicine or many other diseases and, hence, are of diagnostic and/or prognostic relevance. According to one embodiment, the isolated extracellular nucleic acids, such as cell-free DNA, are analyzed to identify and/or characterize a disease.

The analysis/further processing of the isolated nucleic acids can be performed using any nucleic acid analysis/processing method including, but not limited to amplification technologies, polymerase chain reaction (PCR), isothermal amplification, reverse transcription polymerase chain reaction (RT-PCR), quantitative real time polymerase chain reaction (Q-PCR), digital PCR, gel electrophoresis, capillary electrophoresis, mass spectrometry, fluorescence detection, ultraviolet spectrometry, hybridization assays, DNA or RNA sequencing, next generation sequencing, restriction analysis, reverse transcription, nucleic acid sequence based amplification (NASBA), allele specific polymerase chain reaction, polymerase cycling assembly (PCA), asymmetric polymerase chain reaction, linear after the exponential polymerase chain reaction (LATE-PCR), helicase-dependent amplification (HDA), hot-start polymerase chain reaction, intersequence-specific polymerase chain reaction (ISSR), inverse polymerase chain reaction, ligation mediated polymerase chain reaction, methylation specific polymerase chain reaction (MSP), multiplex polymerase chain reaction, nested polymerase chain reaction, solid phase polymerase chain reaction, or any combination thereof. Respective technologies are well-known to the skilled person and thus, do not need further description here.

According to one embodiment, cells comprised in the cells separated by the separation device are analysed. As explained elsewhere herein, the separation device or an element containing the separation device may be removable assembled and therefore, can be easily removed to simplify the analysis of the retained cells and optionally further retained components.

According to one embodiment, cells are analysed by cytological methods. Methods include, but are not limited to histochemical or cytochemical staining such as H&E, Periodic acid schiff (PAS), resorcin fuchsin (RF; elastin stain), sirius red (SR; collagen stain), Gomori (GOM; reticulin stain) and Papanicolaou staining procedure. Furthermore, advanced staining methods such an immunocytology staining or in situ hybridization (ISH) such as FISH (fluorescence), SISH (silver), CISH (chromogenic) may be used. In addition cells can be resuspended and sorted by fluorescence activated cell sorting (FACS) followed by molecular analyses. According to one embodiment, proteins can be extracted and purified from the cells retained in the separation device and analysed e.g. by Western Blot, Enzyme Immunoassay (EIA), Enzyme-linked Immunosorbent Assay (ELISA), proteomics. According to one embodiment, the method comprises preparing exfoliated cells for cytology from the cell sample retained in the separation device. The method according to the present invention allows a subsequent cytological analysis of the retained cells.

According to one embodiment, the separation device is selected such that pathogens, in particular viruses and/or bacteria, are also separated from the flow through allowing extraction, purification and further processing of said captured pathogens that are retained by the separation device, e.g. by the methods described above. For instance, viral nucleic acids and/or bacterial nucleic acids may be purified from the captured viruses and/or bacteria, respectively, and analysed e.g. by amplification technologies, such as polymerase chain reaction (PCR).

The method may also comprise isolating extracellular vesicles from the stabilized cell-depleted biological sample and optionally isolating nucleic acids, such as RNA, from the isolated extracellular vesicles.

### THE METHOD ACCORDING TO THE SECOND ASPECT

According to a second aspect, a method is provided for isolating extracellular nucleic acids from a stabilized cell-depleted biological sample, the method comprising
- stabilizing extracellular nucleic acids in a cell-depleted biological sample obtained from a fluid cell-containing biological sample using the method according to the first aspect; and
- isolating extracellular nucleic acids from the stabilized cell-depleted biological sample.

Details of the method according to the first aspect are described in detail elsewhere herein. Standard methods for isolating extracellular nucleic acids, such as cell-free DNA and/or cell-free RNA were also described above.

In embodiments, the method comprises isolating extracellular vesicles, such as exosomes, from the stabilized cell-depleted biological sample. Suitable methods are known in the art.

### THE KIT AND USE ACCORDING TO THE THIRD AND FOURTH ASPECT

According to a third aspect, a kit is provided for stabilizing extracellular nucleic acids in a cell-depleted biological sample obtained from a fluid cell-containing biological sample, which preferably is a urine sample, said kit comprising
(a) a collection device;
(b) a reception device comprising a stabilizing composition suitable for stabilizing extracellular nucleic acids comprised in the cell-depleted biological sample; and
(c) a separation device that comprises or consists of a separation element that is capable of retaining cells contained in the collected biological sample.

The reception device and the comprised stabilizing composition, as well as the separation device are described elsewhere herein in detail and it is referred to the respective disclosure.

The kit may further comprise
(d) a closing means for closing the collection device and/or
(e) a transfer device for assisting the transfer of fluids from the collection device into the reception device.

The closing means as well as the transfer device are described elsewhere herein in detail and it is referred to the respective disclosure.

In embodiments, the collection device comprises a nuclease inhibitor and/or a cell preserving composition which stabilizes cells and reduces cell lysis. The nuclease inhibitor, preferably a chelating agent such as EDTA, may be comprised in the cell preserving composition. Details are described elsewhere herein in detail and it is referred to the respective disclosure.

The provided kit combines a collection device for collecting a fluid cell-containing biological sample (e.g. urine) with a reception device, such as an evacuated tube, containing a stabilizing composition, preferably a liquid stabilizing solution, for stabilization of extracellular (cell-free) nucleic acids and a separation device for separation, e.g. filtration, of cells and optional further undesired components present in the cell-containing biological sample. The separation device allows the separation of cells from the biological sample before contacting it with the stabilizing composition in the reception device without the need for extra separation measures by clinical personnel or laboratory facilities. Preferably, the separation device is incorporated in the transfer device providing a fluid connection between the collection device and the reception device. The contamination of extracellular nucleic acids by cellular nucleic acids is prevented due to early cell separation and enables a more efficient stabilization of extracellular nucleic acid levels in the cell-depleted biological sample than stabilization alone. Moreover, this allows prolonged storage.

According to a fourth aspect, the present invention is directed to the use of a kit according to the third aspect in a method according to the first and/or second aspect.

### THE USE ACCORDING TO THE FIFTH ASPECT

According to a fifth aspect, the present invention is directed to the use of a separation device that comprises or consists of a separation element that is capable of retaining cells in a method for stabilizing extracellular nucleic acids contained in a fluid cell-depleted biological sample obtained from a fluid cell-containing biological sample, wherein stabilization of the cell-depleted biological sample is performed in a reception device that comprises a stabilizing composition, and wherein said method comprises transferring the cell-containing biological sample through the separation device, thereby separating cells and providing a cell-depleted biological sample and contacting the provided cell-depleted biological sample with the stabilizing composition comprised in the reception device, wherein the transfer is driven by the reception device.

Details of the separation device, the reception device and the comprised stabilizing composition, and the fluid cell-containing biological sample, which preferably is a body fluid, more preferably urine, are described in detail elsewhere herein and it is referred to the respective disclosure.

Also provided as part of the disclosure of the present invention are the below listed items of the present invention.
1. A method for stabilizing extracellular nucleic acids contained in a fluid cell-depleted biological sample obtained from a fluid cell-containing biological sample, which preferably is a urine sample, said method comprising
   (a) obtaining a fluid cell-containing biological sample in a collection device;
   (b) establishing a connection to a reception device which comprises a stabilizing composition suitable for stabilizing extracellular nucleic acids;
   (c) transferring the obtained cell-containing biological sample through a separation device, thereby separating cells and providing a cell-depleted biological sample and contacting the cell-depleted biological sample with the stabilizing composition comprised in the reception device, wherein preferably, the transfer is driven by the reception device.
2. The method according to item 1, wherein the separation device comprises or consists of a separation element that is capable of retaining cells contained in the collected biological sample.
3. The method according to item 1 or 2, wherein cell separation by the separation device occurs by size selection and/or binding characteristics of the separation device.
4. The method according to item 2 or 3, wherein the separation element has one or more of the following characteristics:
   (a) the separation element is provided by a filter or beads;
   (b) the separation element is porous;
   (c) the separation element separates cells and optionally at least one of debris, precipitates and pathogens;
   (d) the separation element allows passage of extracellular vesicles;
   (e) the separation element comprises functional groups.
5. The method according to one or more of items 2 to 4, wherein the separation element is a porous filter, optionally a membrane, sponge or mesh.
6. The method according to one or more of items 1 to 5, wherein the reception device is an evacuated reception device and wherein in (c) the transfer of the cell-containing biological sample through the separation device is driven by the vacuum of the reception device.
7. The method according to one or more of items 1 to 5, wherein the reception device is provided by a syringe and wherein in (c) the transfer of the cell-containing biological sample through the separation device is achieved by drawing up the syringe.
8. The method according to one or more of items 1 to 7, wherein step (a) comprises closing the collection device comprising the fluid cell-containing biological sample with a closing means, preferably a lid.
9. The method according to one or more of items 1 to 8, wherein step (b) establishing a connection to the reception device comprises the use of a transfer device that establishes or contributes to establish a connection between the collection device and the reception device wherein said connection enables the transfer of fluid from the collection device to the reception device.
10. The method according to one or more of items 1 to 9, wherein the transfer device comprises the separation device or wherein the separation device is attached to the transfer device.
11. The method according to item 9 or 10, wherein the transfer device is assembled from at least two elements.
12. The method according to any one of items 9 to 11, wherein the transfer device comprises a connecting element, wherein the connecting element has a distal end directed into the collection device and a proximal end directed to the exterior of the collection device, wherein the proximal end preferably comprises a tip.
13. The method according to item 12, wherein the connecting element is provided by a needle or cannula.
14. The method according to item 12 or 13, wherein the transfer device comprises a transfer element that receives the connecting element.
15. The method according to one or more of items 12 to 14, wherein the connecting element is removably attachable to the transfer element.
16. The method according to one or more of items 12 to 15, wherein the connecting element is comprised in a coupling element that can be removably attached, preferably screwed or clipped, to the transfer element.
17. The method according to one or more of items 10 to 16, wherein a separation device for separating cells in step (c) is comprised in at least one element of the transfer device.
18. The method according to item 17, wherein at least two elements of the transfer device comprise a separation device, optionally wherein each separation device is provided by a filter, optionally wherein the pore size of the filters comprised in the different elements differ from each other.
19. The method according to item 17 or 18, wherein the transfer element comprises a separation device, optionally wherein the separation device comprises or consists of a filter.
20. The method according to any one of items 17 to 19, wherein the connecting element comprises a separation device, optionally wherein the separation device comprises or consists of a filter.
21. The method according to any one of items 17 to 20, wherein the coupling element comprises a separation device, optionally wherein the separation device comprises or consists of a filter.
22. The method according to any one of items 8 to 14, wherein the transfer device or at least an element thereof is connected to the closing means or the collection device, preferably is connected to the closing means which is preferably provided by a lid.
23. The method according to item 22, wherein the transfer device or at least an element thereof is integrated in or removably attachable to the closing means or the collection device, preferably the closing means which is provided by a lid.
24. The method according to one or more of items 8 to 23, wherein the closing means comprises a recess adapted to receive the reception device.
25. The method according to item 24, wherein the recess is provided by an inner protrusion that extends into the collection device when the closing means is mounted to the collection device.
26. The method according to item 24 or 25, wherein a transfer device comprising a connecting element is connected to the closing means, wherein the tip of the connecting element extends into the recess.
27. The method according to any one of items 24 to 26, wherein a transfer element is connected to the bottom of the recess and wherein the transfer element positions the connecting element.
28. The method according to item 26 or 27, wherein the connecting element is comprised in a coupling element that is attached to the transfer element, wherein the connection is preferably removable and preferably provided by screw coupling or clip coupling so that the coupling element is securely but removably attached to the transfer element.
29. The method according to one or more of items 8 to 28, wherein the transfer device comprises a transfer element and a connecting element and is connected to the closing means, wherein the closing means is a lid, wherein the lid has an upper side and a lower side and comprises a recess adapted to receive the reception device, wherein the transfer element of the transfer device is attached to the bottom of the recess on the lower side of the lid and extends into the collection device when the lid is closed, and wherein the connecting element is mounted to the bottom of the recess, preferably via the transfer element, wherein the proximal end of the connecting element comprising the tip is positioned within and extends into the recess, preferably wherein the connecting element is provided by a needle or a cannula.
30. The method according to item 29, wherein the connecting element is comprised in a coupling element that is attached, optionally screwed or clipped, to the transfer element.
31. The method according to one or more of items 8 to 30, wherein the transfer device or at least an element thereof is removably attachable to the collection device or the closing means of the collection device.
32. The method according to item 31, wherein the transfer device is removably attachable to the collection device or the closure means of the collection device and wherein the transfer device comprises a transfer element and a connecting element,
   wherein at least an element of the transfer device is inserted into the collection device through an access port, wherein the access port is positioned in the closing means,
   wherein the transfer element of the transfer device provides a recess, preferably a cup-like receptable, adapted to receive the reception device, wherein the cup-like receptable is positioned on the outer side of the access port,
   and wherein the proximal end of the connecting element comprising the tip is positioned within the cup-like receptable.
33. The method according to item 32, wherein the access port is closed by a cover means, preferably wherein the cover means is an elastomeric self-sealing material.
34. The method according to one or more of items 1 to 33, wherein the separation device is comprised in the reception device.
35. The method according to one or more of items 1 to 34, wherein the cell-containing biological sample is a body fluid, optionally selected from urine, mouth washes, saliva, sputum, bronchial lavages, blood, plasma, serum, edema, pleural effusions, breast milk, tears, sweat, cerebrospinal fluid, synovial fluid, semen, vaginal fluid, ascitic fluid and amniotic fluid.
36. The method according to item 35, wherein the cell-containing biological sample is urine.
37. The method according to one or more of items 1 to 36, wherein the stabilizing composition comprised in the reception device protects extracellular nucleic acids comprised in the cell-depleted biological sample from degradation.
38. The method according to item 37, wherein the stabilizing composition comprises a nuclease inhibitor.
39. The method according to item 38, wherein the nuclease inhibitor is a chelating agent, preferably EDTA.
40. The method according to any one of items 37 to 39, wherein the stabilizing composition comprises an additive for cell and/or cellular component stabilization, preferably at least one volume excluding polymer.
41. The method according to item 40, wherein the volume excluding polymer is a poly(oxyethylene) polymer, preferably polyethylene glycol.
42. The method according to item 41, wherein the stabilizing composition comprises a high molecular weight poly(oxyethylene) polymer and a low molecular weight poly(oxyethylene) polymer.
43. The method according to one or more of items 37 to 42, wherein the stabilizing composition comprises one or more of the following
   (i) a preservative for inhibiting bacterial growth;
   (ii) a caspase inhibitor;
   (iii) a primary, secondary or tertiary amide; and/or
   (iv) a buffering agent.
44. The method according to one or more of items 1 to 43, wherein the stabilizing composition is a liquid or dried composition.
45. The method according to one or more of items 37 to 44, wherein the stabilizing composition is a liquid that has a pH that lies in the range of 4 to 9.5, such as 4.3 to 9.5, 4.5 to 9.5, 4.7 to 9.5, 5 to 9.5, 5.5 to 9.5, 6 to 9.5, 6.5 to 9.5, 6.7 to 9.0, 7.0 to 9.0 or 7.2 to 9.0, preferably 7.2 to 7.8, such as 7.5.
45. The method according to one or more of items 1 to 44, wherein the stabilizing composition is a liquid composition and the reception device is designed so that the liquid composition is contacted with the received cell-depleted biological sample, preferably a cell-depleted urine sample, in a volumetric ratio selected from 1:3 to 1:10, preferably 1:5 to 1:10, more preferred 1:6 to 1:7.
46. The method according to one or more of items 39 to 45, wherein the final concentration of the chelating agent, preferably EDTA, in the stabilized cell-depleted biological sample lies in a range of 2mM to 100mM, preferably 10mM to 50mM, more preferred 20mM to 50mM.
47. The method according to one or more of items 1 to 46, wherein the collection device comprises the separation device for separating cells.
48. The method according to one or more of items 1 to 47, wherein the collection device comprises a nuclease inhibitor and/or a cell preserving composition.
49. The method according to item 48, wherein the cell preserving composition is a liquid or dried composition, preferably a spray-dried composition.
50. The method according to item 48 or 49, wherein the cell preserving composition stabilizes cells and reduces cell lysis.
51. The method according to any one of items 48 to 50, wherein the cell preserving composition comprises a volume excluding polymer and/or an osmotic agent.
52. The method according to item 51, wherein the cell preserving composition comprises (i) at least one compound selected from a poly(oxyethylene) polymer, preferably polyethylene glycol (PEG), glycerol, trehalose, dextrans and derivatives thereof, optionally selected from dextran sulfate and dextran acetate, and hydrophilic polymers optionally selected from polyvinyl alcohol, polyvinyl acetate and polyvinyl sulfate and/or (ii) at least one compound selected from sugars, sugar alcohols and salts.
53. The method according to one or more of items 47 to 52, wherein the cell preserving composition comprises a nuclease inhibitor, preferably a chelating agent, more preferably EDTA.
54. The method according to one or more of items 1 to 53, wherein the stabilization composition comprised in the reception device is a liquid composition that comprises:
   - a chelating agent, preferably EDTA;
   - at least one poly(oxyethylene) polymer, preferably polyethylene glycol;
   - optionally a buffering agent.
55. The method according to one or more of items 1 to 54, wherein the stabilization composition comprised in the reception device comprises:
   - a chelating agent, preferably EDTA, in a concentration of 150mM up to the solubility limit;
   - a high molecular weight poly(oxyethylene) polymer, preferably polyethylene glycol, having a molecular weight in the range of 1500 to 40000, such as 3000 to 25000;
   - a low molecular weight poly(oxyethylene) polymer, preferably polyethylene glycol, having a molecular weight in the range of 100 to 1000, such as 200 to 700; and
   - optionally a buffering agent.
56. The method according to one or more of items 1 to 55, the method comprising storing and/or shipping the reception device comprising the stabilized cell-depleted biological sample.
57. The method according to one or more of items 1 to 56, the method comprising isolating extracellular nucleic acids, such as extracellular DNA and/or extracellular RNA from the stabilized cell-depleted biological sample.
58. The method according to one or more of items 1 to 57, the method comprising isolating extracellular vesicles from the stabilized cell-depleted biological sample and optionally isolating nucleic acids, such as RNA, from the isolated extracellular vesicles.
59. The method according to one or more of items 1 to 58, the method comprising processing the separated cells.
60. The method according to item 59, wherein processing the separated cells comprises (i) analysing the separated cells and/or (ii) extracting nucleic acids from the separated cells.
61. The method according to item 59 or 60, wherein the separation device or an element containing the separation device is removable.
62. A method for isolating extracellular nucleic acids from a stabilized cell-depleted biological sample, the method comprising
   - stabilizing extracellular nucleic acids in a cell-depleted biological sample obtained from a fluid cell-containing biological sample using the method according to items 1 to 56; and
   - isolating extracellular nucleic acids from the stabilized cell-depleted biological sample.
63. The method according to item 62, wherein the cell-containing biological sample is a urine sample.
64. The method according to item 62 or 63, wherein the extracellular nucleic acid is extracellular DNA.
65. A kit for stabilizing extracellular nucleic acids in a cell-depleted biological sample obtained from a fluid cell-containing biological sample, which preferably is a urine sample, said kit comprising
   (a) a collection device;
   (b) a reception device comprising a stabilizing composition suitable for stabilizing extracellular nucleic acids comprised in the cell-depleted biological sample; and
   (c) a separation device that comprises or consists of a separation element that is capable of retaining cells contained in the collected biological sample.
66. The kit according to item 65, wherein the kit further comprises
   (d) a closing means for closing the collection device and/or
   (e) a transfer device for assisting the transfer of fluids from the collection device into the reception device.
67. The kit according to item 65 or 66, wherein the reception device has the characteristics as defined in one or more of the above items, in particular in any one of items 6, 7 and 34.
68. The kit according to any one of items 65 to 67, wherein the kit comprises a transfer device and wherein the transfer device has the characteristics as defined in one or more of the above items, in particular in any one of items 9 to 23 and 26 to 32.
69. The kit according to any one of items 65 to 68, wherein the separation device has the characteristics as defined in one or more of the above items, in particular in any one of items 2 to 5 and 17 to 21.
70. The kit according to one or more of items 65 to 69, wherein the kit comprises the closing means and wherein the closing means has the characteristics as defined in one or more of the above items, in particular in any one of items 8 and 24 to 33.
71. The kit according to one or more of items 65 to 70, wherein the stabilizing composition comprised in the reception device has the characteristics as defined in one or more of the above items, in particular in any one of items 37 to 46 and 54 to 55.
72. The kit according to one or more of items 65 to 71, wherein the collection device has one or more of the following characteristics:
   i) it has the characteristics as defined in one or more of the above items, in particular in any one of items 47 to 53;
   ii) it comprises a nuclease inhibitor, preferably a chelating agent such as EDTA;
   iii) it comprises a cell preserving composition which stabilizes cells and reduces cell lysis; and/or
   iv) it comprises a closing means as defined in item 70.
73. The kit according to one or more of items 65 to 72, wherein the collection device comprises a cell preserving composition that has the characteristics as defined in one or more of items 49 to 53.
74. Use of a kit according to one or more of items 65 to 73 in a method according to one or more of items 1 to 64.
75. Use of a separation device that comprises or consists of a separation element that is capable of retaining cells in a method for stabilizing extracellular nucleic acids contained in a fluid cell-depleted biological sample obtained from a fluid cell-containing biological sample, wherein stabilization of the cell-depleted biological sample is performed in a reception device that comprises a stabilizing composition, and wherein said method comprises transferring the cell-containing biological sample through the separation device, thereby separating cells and providing a cell-depleted biological sample and contacting the provided cell-depleted biological sample with the stabilizing composition comprised in the reception device, wherein the transfer is driven by the reception device.

As is apparent from the above disclosure and the examples provided below, the present invention provides improved methods and kits for processing and stabilizing a fluid cell-containing biological sample, in particular a urine sample, compared to prior art methods. In particular, a more sterile, simpler, faster, and more convenient processing of fluid cell-containing biological samples is provided. Furthermore, the safety is increased since no open handling of the potentially infectious sample is required. In addition, less equipment is needed and less processing steps are required due to simultaneous cell separation (no centrifugation required) and transfer of the sample into the reception device. In addition, contamination and dilution of the target analytes, preferably extracellular nucleic acids (e.g. cfDNA, cfRNA), is prevented or significantly reduced since contaminating components present in the cell-containing biological sample are retained in the separation device during transfer from the collection device into the reception device. This provides more reliable results when target extracellular nucleic acids are quantified. Moreover, this allows separate analysis of the extracellular nucleic acids and of the cells and optional further retained components providing more information on the overall health status of the subject. The so obtained stabilized cell-depleted urine sample can be stored at room temperature, and even elevated temperatures of up to 40°C without severe extracellular cell-free nucleic acid degradation or contamination by intracellular nucleic acids. An instant preservation of urine cell-free nucleic acids is provided by the methods and kits of the present invention allowing a simple, fast and safe cell separation and stabilization workflow that can be conducted by the patient, e.g. at the doctor's office, in a clinic or at home, without the need for clinical personnel or laboratory facilities and equipment (e.g. centrifuge).

This invention is not limited by the exemplary methods and materials disclosed herein, and any methods and materials similar or equivalent to those described herein can be used in the practice or testing of embodiments of this invention. Numeric ranges are inclusive of the numbers defining the range. The headings provided herein are not limitations of the various aspects or embodiments of this invention which can be read by reference to the specification as a whole.

Unless the context indicates otherwise, percentage values indicated herein refer to (w/v) in case of solid compounds contained in a liquid mixture or composition and to (v/v) in case of liquid compounds contained in a liquid mixture or composition such as e.g. the mixture resulting from contacting the stabilizing composition with the cell-depleted biological sample.

As used in the subject specification, the singular forms "a", "an" and "the" include plural aspects unless the context clearly dictates otherwise. Thus, for example, reference to an "agent", "additive", "compound" and the like includes single entities and combinations of two or more of such entities. Reference to "the disclosure" and "the invention" and the like includes single or multiple aspects taught herein; and so forth. Aspects taught herein are encompassed by the term "invention".

The term "solution" as used herein in particular refers to a liquid composition, preferably an aqueous composition. It may be a homogenous mixture of only one phase but it is also within the scope of the present invention that a solution comprises solid additives such as e.g. precipitates, in particular of contained chemicals such as stabilizing agents.

The terms "stabilization composition" and "stabilizing composition" are used as synonyms herein.

The terms "filter" and "filter medium" are used as synonyms herein.

As used herein, the term "comprising" is to be construed as encompassing both "including" and "consisting of", both meanings being specifically intended, and hence individually disclosed embodiments in accordance with the present invention. According to one embodiment, subject matter described herein as comprising certain steps in the case of methods or as comprising certain ingredients in the case of compositions, solutions and/or buffers refers to subject matter consisting of the respective steps or ingredients. It is preferred to select and combine preferred embodiments described herein and the specific subject-matter arising from a respective combination of preferred embodiments also belongs to the present disclosure.

### EXAMPLES

It should be understood that the following examples are for illustrative purpose only and are not to be construed as limiting this invention in any manner.

### Abbreviations

- ccfDNA:: circulating, cell-free DNA
- cfDNA:: cell-free DNA
- gDNA:: genomic DNA
- PEG:: Polyethylene glycol
- EDTA:: Ethylenediaminetetraacetic acid
- DMPA:: Dimethylpropionamide
- DEGMEA:: Diethlyeneglycol-monoethylether-acetate
- PBS:: Phosphate buffered saline

### Example 1: Collection and processing of urine samples with a system comprising a collection device, a reception device and a filter device

**Figure 1** shows a comparison of the common workflow for cell-free DNA (cfDNA) isolation from urine to the workflow of the present invention.

### 1. Common urine collection and processing workflow:

In common urine collection and processing workflows, first, urine is collected in a urine collection cup (see **Figure 1A**). Subsequently, a stabilizing solution is added to the urine sample. This step has several disadvantages. First, it requires handling of urine and stabilizing solution. Furthermore, laboratory personnel are required. Moreover, the ratio of urine to stabilizing solution is not defined. After stabilization of the urine, the stabilized urine samples need to be aliquoted. Again, handling of the urine-stabilizing solution-mixture is required. Cells present in the urine-stabilization mixture bear the risk for gDNA release that contaminates cfDNA and lysed cells may also release nucleases that can degrade extracellular nucleic acids. Afterwards, the cells present in the urine samples need to be separated. This is usually achieved by centrifugation or filtration of the stabilized sample, which requires laboratory personnel as well as additional equipment. Only after these multiple steps, extracellular nucleic acids, such as cfDNA, can be isolated from the processed urine sample.

### 2. Urine collection and processing workflow according to the invention:

In contrast, the kits, devices and methods of the present invention provide a simple, fast and safe workflow (see **Figure 1B**). Furthermore, sterility can be provided during handling of the urine sample since after urine collection no open handling is required.

The urine is collected in a collection device (e.g. a urine collection cup). The collection device is closed by a lid that allows to establish a connection to a reception device, which preferably is an evacuated tube. The tube can be connected to a needle that is comprised in the lid which is connected to a straw (transfer element) which extends into the collection container and is in contact with the collected specimen, here urine. The needle may be embedded in a deepening/recess in the lid that is sealed or otherwise covered by a suitable cover means, such that the risk of injury is reduced.

For the aspiration of urine into the reception device, the cover means is removed, and the evacuated tube is pushed into the recess of the lid where the cap of the evacuated tube is pierced by the needle. The vacuum of the reception device enables the automatic inflow of the urine from the collection cup through the straw into the evacuated tube. The transfer of the urine is driven by the reception device, in the illustrated embodiment by the vacuum of the evacuated tube.

Cell separation occurs during the process of urine transfer from the collection cup into the evacuated tube. As disclosed herein, there are multiple options to position the separation device, such as a filter, to achieve cell separation during the transfer of the biological sample from the collection device into the reception device. For example, a separation element, such as a filter, can be advantageously incorporated into the straw (transfer element) that reaches to the bottom of the container. When the evacuated tube is connected to the closed collection device and pierced by the needle, urine is sucked through the straw by the vacuum and forced during the transfer into the evacuated tube to pass the filter element that is contained in the straw. The filter retains the cells, and thus the cellular DNA contained in the cells, and optionally further undesired components such as debris that shall be prevented from passing into the evacuated tube where the obtained cell-depleted biological sample is stabilized by the stabilizing composition. Alternatively, or additionally, the separation device, such as a filter element, may be part of the reception device, and may e.g. be placed in the cap of the evacuated tube. The separation device may also be located within the collection device. The separation device may also be separately connected between the collection cup and the evacuated tube.

The inflow of the biological sample into the evacuated tube is terminated once the desired volume has been aspired by the vacuum. The filled reception device can then be removed. As is described elsewhere herein, the cap of the tube can be automatically sealed so that the collected sample cannot spill from the tube. To ensure good mixing of the stabilizing composition with the cell-depleted biological sample, the reception device with the collected sample can be inverted several times. The reception device which comprises the stabilized sample can then be processed further, e.g. stored, shipped or directly processed to isolate extracellular nucleic acids, such as cfDNA, from the stabilized sample. The invention also enables the long-term storage of the stabilized cell-depleted samples by using a suitable stabilizing composition. As disclosed herein, the stabilizing composition is preferably non-crosslinking and does not modify the extracellular nucleic acids that are comprised in the stabilized cell-depleted biological sample.

Moreover, also the separated cells may be further processed, e.g. by further analysis (cytology), cultivation or further common downstream applications. Moreover, nucleic acids such as gDNA or mRNA may be isolated from the retained cells.

The collection device and/or the closing means that is mounted to the collection device for closure (lid), may comprise a cell preserving composition as is described elsewhere herein. Such cell preserving composition may assist in preventing cell disruption, e.g. by protecting the cells from damage by shear forces, thereby reducing or preventing cell disruption and, thus, the release of gDNA and moreover nucleases from the cells. In embodiments, the same stabilizing composition is comprised in the reception device and the collection device.

Advantages of the workflow according to the present invention are among others that no open handling of urine and stabilizing solution is required, the cell separation can be performed by the donor due to easy handling of the devices, the provided workflow is fast, simple and requires less equipment and disposables, and there is a reduced risk for gDNA contamination due to separation of cells and optionally further undesired components early during processing of the biological sample.

The kits, devices and methods are in particular suitable for the processing of urine, but are also suitable for other biological fluids, such as mouth washes, saliva, sputum, bronchial lavages, edema, pleural effusions, blood, plasma or serum. Moreover, the separation device, such as the filter element, can be adjusted to different analyte targets and to the components that shall be removed from the sample. For instance, the pore size of the filter and/or its surface chemistry can be adjusted accordingly.

The kits, devices, and methods according to the present invention allow home and point-of-care collection and processing of fluid biological samples, such as urine, by providing an easy-to-use system that combines the reduction of cellular nucleic acid contamination due to filtration and prevention of cell free nucleic acid degradation due to stabilization of the filtered samples.

### Example 2: Effect of urine stabilization with a stabilizing solution on nucleic acid yield

### 1. Female urine collection and spike-in of cell-free male urine:

The second urine of the day was collected from 12 female donors. As much urine as possible was collected in two urine cups per donor.

Cell-free male urine (unstabilized pool of urine samples from 12 donors, second morning urine) was prepared by centrifugation and was frozen. The prepared cell-free male urine was thawed at room temperature 1 hour prior to female urine collection and spiked into the female urine. The ratio of donated female urine to male spike-in urine was approximately 10:1. The total volume of spike-in urine amounted to approximately 105 ml.

### 2. Urine stabilization:

For stabilization of urine samples either a stabilizing solution was prepared according to the teachings of WO 2020/141187 A1 (see in particular Table 1 therein), commercially available PAXgene^{®} Blood ccfDNA tubes (PreAnalytiX) or EDTA tubes were used.

The spike-in urine samples were aliquoted into 50 ml tubes (preparation of 25.3 ml for unstabilized sample and 22 ml for stabilized sample) and either kept unstabilized (no stabilizing reagent added) or mixed with the stabilizing solution of WO 2020/141187 A1 that can be advantageously used as stabilization composition according to the present invention. 22 ml of urine were mixed with 3.3 ml of stabilizing solution (ratio 6.67:1) that was prepared according to WO 2020/141187 A1. For mixture of the urine samples with the stabilization reagents within the PAXgene^{®} ccfDNA tubes and EDTA tubes, 50 ml urine was aliquoted in a urine cup and aspired with the respective tubes to a total volume of 10 ml. For thorough mixing of urine and stabilization reagents, all samples were inverted five times. Duration from collection until stabilization was less than 15 min.

The stabilized urine samples were divided into two samples for two different time points (see below) and aliquots of 11.5 ml were prepared. The samples were either directly analyzed (T0) or stored for three days prior to analysis (T3) at ambient temperature (uncontrolled room temperature).

The urine samples stabilized with the stabilizing solution according to the teachings of WO 2020/141187 A1 contained a caspase inhibitor (Q-VD-Oph), PEG10000, PEG300, ProClin^{™}300, and a buffer (see WO 2020/141187 A1, Table 1 on p. 48 and 49).

### 3. Cell-free DNA isolation and quantitative, real-time PCR:

Female cells were separated from the stabilized urine samples by centrifugation at 1900xg for 15 min. The supernatant was transferred to a new 15 ml tube and centrifuged at 1900xg for 10 min. Afterwards, the supernatant was recovered for cfDNA isolation.

Cell-free DNA from 4.8 ml or 9.6 ml, respectively, stabilized or unstabilized urine samples was isolated using the QIAsymphony PAXgene@ Blood ccfDNA Kit (QIAGEN) protocol either directly after donation (T0) or three days after donation (T3) (storage at ambient temperature (uncontrolled room temperature)). DNA was eluted to reach a final eluate volume of 60 µl.

Subsequently, the Y chromosome-specific gene DYS14 was detected by quantitative, real-time PCR using 7 µl of eluate in a 20 µl assay volume. As no male cells were included in the processed urine, changes in DYS14 levels were caused by degradation only and not by release of cellular DNA.

### Discussion:

The results presented in **Figure 2** demonstrate that unstabilized urine samples showed high DYS14 DNA degradation after three days of storage. In contrast, the urine samples stabilized with the stabilizing solution prepared according to the teachings of WO 2020/141187 A1 showed a mean degradation of only about 5% and, thus, were considered very stable. With PAXgene^{®} ccfDNA and EDTA tubes, degradation compared to unstabilized urine samples was reduced but significantly higher compared to the urine samples stabilized with the stabilizing solution of the present invention. Thus, the stabilizing solution prepared according to WO 2020/141187 A1 achieved the highest stability of DYS14 DNA.

The quantities used in this example are for illustrative purpose only and volumes may be adjusted in consideration of the advantageous ratios and concentrations described above.

### Example 3: Effect of urine filtration subsequent to urine collection on nucleic acid yield

During storage of urine samples cells contained in the samples release gDNA that contaminates the cfDNA and impairs downstream analyses. To reduce the effect of gDNA contamination, urine samples may be filtered to remove cells prior to storage.

### 1. Female urine collection and spike-in of cell-free male urine:

Female urine samples were collected, and cell-free male urine was spiked-in in a ratio as described in Example 2. The ratio of donated female urine to male spike-in urine was approximately 6:1. The total volume of spike-in urine amounted to approximately 110 ml.

### 2. Urine filtration:

Subsequently, the total volume of spiked urine was divided into two samples. One of the urine samples was filtered using a 0.8 µm syringe filter.

### 3. Urine stabilization:

The filtered and unfiltered urine samples were each divided into further two samples. One of the filtered and one of the unfiltered samples were each transferred to a 50 ml tube containing the stabilization reagent that was prepared according to WO 2020/141187 A1 (see above). 22 ml of filtered or unfiltered urine were added to 3.3 ml of stabilizing solution (ratio 6.67:1) and the samples were inverted five times for thorough mixing. Duration from collection until stabilization was less than 30 min.

Next, 11.5 ml of the samples of each condition (i.e. (i) unstabilized and unfiltered, (ii) unstabilized and filtered, (iii) stabilized and unfiltered, and (iv) stabilized and filtered) were aliquoted.

### 4. Cell-free DNA isolation and quantitative, real-time PCR:

Separation of female cells, isolation of cfDNA from 9.6 ml processed urine samples and quantitative, real-time PCR were performed as described in Example 2 with the difference that 18S was analyzed as a marker for total DNA.

### Discussion

In this example the effect of urine filtration prior to stabilization was analyzed (see **Figure 3A**). In case the collected urine is directly mixed with a stabilizing solution the stabilized urine samples contain cells and cellular components which release gDNA during storage. In contrast, including a step of filtration directly after collection separates the cells and optionally further components, and thus prevents gDNA contamination. The addition of a stabilizing composition such as a solution to the filtered urine samples reduces degradation of the target cfDNA during storage. Moreover, the cells and the optionally further components retained in the filter medium may be recovered and analyzed separately.

The stabilized unfiltered urine samples, which were prepared according to Example 1, showed a two-fold increase of 18S levels after three days of storage indicating gDNA release (see **Figure 3B**). In contrast, the 18S levels were kept relatively stable when the urine samples were additionally filtered prior to stabilization which indicates an efficient reduction of cellular DNA release. This demonstrates the important advantages of direct cell separation in accordance with the teachings of the present invention. As disclosed herein, there are numerous advantageous options to position a separation device, such as a filter element, to achieve cell separation, in particular during the transfer into the reception device.

In the two stabilized samples, either filtered or unfiltered, no degradation of DNA occurred. On the contrary, neither in the unstabilized, unfiltered nor the unstabilized, filtered urine samples degradation of DNA could be prevented.

The quantities used in this example are for illustrative purpose only and volumes may be adjusted in consideration of the advantageous ratios and concentrations described above. The results of this example demonstrate the advantages of the kits, devices and methods of the present invention which provide not only stabilization of target analytes, in particular cfDNA, in urine samples, but additionally prevent contamination by undesired components present in urine, such as cellular nucleic acids.

### Example 4: Assembly of a collection device, a reception device with a stabilizing composition and a separation device

In this example suitable and preferred embodiments of the present invention are described.

A fluid cell-containing biological sample (e.g. urine, mouth washes or saliva) is obtained into a collection device, which is directly closed by a closing means, preferably a lid, after collection. The cell-containing biological sample is then transferred into the reception device which preferably is an evacuated tube. During transfer, cells are separated from the flowthrough by a separation device thereby providing a cell-depleted biological sample. According to a preferred embodiment, the separation device, which in embodiments is provided by a filter element, is comprised in a transfer device, which provides a connection for fluids between the collection device and the reception device. Different potential assemblies and embodiments are discussed in detail in the following.

### Integrated transfer device comprising the separation device

According to one embodiment shown in **Figure 4**, the lid (11) comprises a recess (17) dimensioned for receiving the reception device (15), which preferably is an evacuated tube. A connecting element (14), preferably a needle or a cannula, is mounted to the bottom of the recess (17) and positioned by a transfer element (16) that is in the shown embodiment molded to and thus part of the lid. The transfer element (16), here in form of an open hollow tube, is attached to the lower side of the recess (17) of the lid (11) and extends into the collection device (10). The transfer tube (16) and the connecting element (14) constitute in the shown embodiment the transfer device according to the present invention, which - according to this embodiment - is permanently attached to the lid (11) of the collection device (10) (i.e. integrated transfer device). The distal end of the connecting element (14a) extends into the collection device (10). When the reception device (15) is inserted into the recess (17) the cap (15a), e.g. a self-sealing stopper, of the evacuated reception device is pierced by the tip at the proximal end of the connecting element (14b). Due to the vacuum present in the interior of the reception device (15), the cell-containing biological sample, e.g. urine, is drawn up through the connecting element (14) into the reception device (15). During passage from the collection device (10) into the reception device (15) the cells contained in the cell-containing biological sample are separated by the separation device that is included in the transfer device (not shown), and which preferably is a filter. The separation device may be included in the connecting element (14) or may be attached as separate element (e.g. a filter disk) to the distal end of the connecting element (14a). The separation device/separation element retains the cells whereby a cell-depleted biological sample is provided. The reception device (15) contains a stabilizing composition, preferably a liquid stabilizing solution. The cell-depleted biological sample contacts the stabilizing composition when it enters the interior of the reception device (15). Once the desired volume of cell-depleted biological sample is transferred (e.g. driven by the vacuum of the reception device), the reception device (15) can be removed from the tip of the connecting element (14b) and the self-sealing lid (15a) prevents leakage of cell-depleted biological sample from the reception device (15). In a preferred embodiment, the volumetric ratio is set and lies in the range of 1:5 to 1:7 (see also Examples 2 and 3 above). For ensuring thorough mixing of the stabilizing solution and the cell-depleted biological sample, the reception device (15) may be inverted several times and a stabilized cell-depleted biological sample, e.g. a stabilized cell-depleted urine sample, is provided. Subsequently, the stabilized cell-depleted biological sample can be stored at room temperature e.g. for at least three days prior to further processing and analysis. Alternatively, the stabilized cell-depleted biological sample can be stored at reduced temperatures, e.g. at 4°C in the refrigerator, also for prolonged time periods. Moreover, the method of the present invention provides storing at elevated temperatures, such as up to 30°C or up to 40°C. According to one embodiment, the collection device (10) comprises a cell preserving composition. The cell preserving composition preferably protects the cells from damage by shear forces acting during the passage through the transfer device. This advantageously allows to further reduce the contamination and dilution of extracellular nucleic acids in the flowthrough with intracellular nucleic acids released by cell damage.

In the following two assemblies and the position of the separation device according to the present invention are described with reference to **Figures 4A** and **4B****.**

According to one embodiment shown in **Figure 4A****,** the distal end of the connecting element (14a) extending into the collection device (10) nearly reaches the bottom of the collection device (10) such that the opening of the distal end of the connecting element (14), preferably a needle or a cannula, is covered by the fluid cell-containing biological sample. In this embodiment, the part of the connecting element (14) extending into the collection device (10) is longer than the transfer element (16). The separation device, which preferably is provided by a filter (not shown), is comprised in the interior of the connecting element (14) such that the cells and optionally further undesired components present in the cell-containing biological sample are separated from the flowthrough during passing through the connecting element (14) into the reception device. Thus, the separation device may be included in the connecting element (14) (not shown in **Figure 4A****).** According to one embodiment, the separation device can be removed from the connecting element (14) after the biological sample has been transferred from the collection device (10) into the reception device (15) and the separated cells, and optional further undesired components, can be extracted and analysed as described above.

According to the embodiment shown in **Figure 4B****,** the distal end of the transfer element (16) that extends into the collection device (10) nearly reaches the bottom of the collection device (10) such that the opening of the distal end of the transfer element (16), preferably a straw (see e.g. **Figures 1** and **3A****),** is covered by the fluid cell-containing biological sample. In this embodiment, the transfer element (16) is longer than the part of the connecting element (14) that extends into the collection device (10) so that the opening at the distal end of the connecting element (14a) is in the interior of the transfer element (16). The separation device (18), which preferably is provided by a filter, is comprised in the interior of the transfer element (16) in front of the opening of the distal end of the connecting element (14a) such that cells and optionally further undesired components present in the cell-containing biological sample are separated from the flowthrough during passage through the transfer element (16) into the reception device. Thus, the separation device (18), e.g. a filter element, is included in the transfer element (16) (see **Figure 4B****).** According to one embodiment, the distal end of the transfer element (16) is enlarged to provide a larger reception for the separation element, e.g. a filter. E.g. the distal end of the transfer element may be enlarged in a disk-shaped manner thereby providing a broader area for the filter and reducing the shear forces for the cells during cell separation. Furthermore, and as is illustrated in **Figure 4C****,** the separation device may also be provided as separate device (19) with a housing for the separation element, which e.g. is a filter, that is mounted, e.g. screwed or clipped, to the transfer device or an element thereof.

According to one embodiment, the separation device (18/19) is removed from the transfer element (16) after the biological sample has been transferred from the collection device (10) into the reception device (15) and the cells, and optional further retained components, can be extracted and analysed as described above. According to a further embodiment, the transfer element (16) and the connecting element (14) each comprise a separation device. E.g. both the transfer tube (16) and the connecting element (14) may comprise a filter. According to one embodiment, the pore size of the filters varies. For instance, the pore size of the filter comprised in the transfer element (16) is larger than the pore size of the filter comprised in the connecting element (14). Consequently, larger cells are retained in the filter of the transfer element (16) while smaller components, such as smaller cells, pathogens, subcellular components and/or further particles such as debris, are retained in the filter of the connecting element (14). According to one embodiment, the filter can be removed from the transfer element (16) and/or the connecting element (14) allowing for separate extraction and analysis of the retained components. Likewise, the separation devices comprised in the transfer element (16) and the connecting element (14) may be of different types, e.g. one comprises a filter and the other may comprise beads. Different suitable combinations are within the scope of the present disclosure and discussed elsewhere herein.

Optionally, the bottom of the collection device (10) has a reception (12), which can be provided as deepening or heightened rim, into which the distal end of the transfer element (16) and/or the distal end of the connecting element (14a) extends. This is particularly advantageous to reduce volume losses during sample processing.

In **Figure 4** the recess (17) is located in the lid (11) of the collection device (10). However, it is also within the scope of the present invention that the recess is located in the wall or the bottom of the collection device (10). The same principles as described above also apply in such an assembly. For instance, the recess (17) and the attached transfer device with the transfer element (16) and the connecting element (14) may be located in the lower part of the wall of the collection device (10) such that the opening of the connecting element (14) or of the transfer element (16), respectively, is covered by the cell-containing biological sample. Alternatively, in case the recess (17) and the attached transfer device with the transfer element (16) and the connecting element (14) are located in the bottom of the collection device (10), the distal part of the connecting element (14) or of the transfer element (16), respectively, extending into the collection device may be shorter. Moreover, the collection device (10) may have a first bottom comprising the recess (17) and a second bottom above the first bottom that defines the interior in which the cell-containing biological sample is contained.

**Figure 4C** illustrates a further embodiment wherein the separation device (19) comprises a separation element (18), in the illustrated embodiment a porous filter, in a housing. The housing of the separation device may have an open bottom or a bottom with openings to allow the flow of the cell-containing biological sample through the separation device (see arrows). The top of the separation device can be mounted to the transfer device, e.g. the distal end of the transfer element (16) or the distal end of connecting element **(****Figure 4C** illustrates the distal end of the transfer element (16)). The separation device preferably provides an enlarged cross section compared to the element of the transfer device to which it is connected (e.g. compared to the transfer element (16)). This enlarged cross section of the separation device advantageously reduces the shear forces that are applied to the cells during cell separation and further reduces the risk of cell damage and thus release of intracellular nucleic acids and nucleases. The separation device can also be removed after use and the retained cells further processed as described elsewhere herein.

### Removably attachable transfer device comprising the separation device

According to the embodiment shown in **Figures 5A** and **5B****,** the transfer element (116) and the connecting element (114), forming the transfer device, are removably attachable to the lid (111) of the collection device (110). The upper surface of the lid (111) includes an access port (120) extending therethrough. The access port (120) provides communication to the interior of the collection device (110) through the lid (111). To sealably close the access port (120) a self-sealing cover means (130) is provided, e.g. a self-sealing elastomeric stopper, which may be attached to the lid (111) and interposed within access port (120). The cover means (130) permits insertion of a transfer device - comprising a connecting element (114) and a transfer element (116) - therethrough. Moreover, the cover means (130) provides for sealed engagement with the transfer device upon insertion and also provides for self-sealing upon removal of the transfer device therefrom. The transfer device formed by the transfer tube (116) and the connecting element (114), preferably a needle or cannula, permits the transfer of the cell-containing biological sample from the collection device (110) into the reception device (115), preferably an evacuated tube, that comprises the stabilizing composition according to the present invention. The distal end of the connecting element (114a) can be inserted through the access port (120) with the cover means (130) into the collection device (110), where it nearly reaches the bottom when it is inserted. The proximal end of the connecting element (114b) is positioned externally of the collection device (110) and comprises a piercing tip which is designed for piercing engagement through the cap (115a) of the reception device. The connecting element (114) thus provides fluid communication between the collection device (110) and the reception device (115). Furthermore, the connecting element (114) may comprise the separation device, such as a filter or a separate device having a housing that comprises the separation element may be attached to the distal end of the connecting element (114) (not indicated in **Figure 5**). The transfer element may be provided by a cup-like receptable which provides for the accommodation of the top of the reception device (115). Hence, once the cell-containing biological sample, e.g. urine or saliva, is obtained in the collection device (110), the transfer device can be inserted through the access port (120) and the cover means (120) (see also dashed line in **Figure 5A**). Next, the reception device (115) can be inserted into the transfer element (116) such that the lid of the reception device (115a) is pierced by the tip of the connecting element (114). This establishes a fluid communication between the collection device (110) and the reception device (115) and the cell-containing sample is drawn up through the connecting element (114) by the vacuum of the reception device. Thereby, the sample passes the separation device allowing separation of cells and optionally further undesired components from the flowthrough and providing a cell-depleted biological sample that enters the reception device (115) and contacts the stabilizing composition according to the present invention. Once the desired amount of sample is transferred into the reception device (115), the reception device (115) can be removed from the transfer tube (116). The self-sealing lid (115a) prevents leakage. To ensure thorough mixing of the cell-depleted biological sample and the stabilizing composition the reception device (115) can be carefully inverted. Subsequently, the stabilized cell-depleted biological sample can be stored at room temperature for at least three days or at lower temperatures for a longer time period (e.g. 4°C) prior to further downstream processing and analysis.

According to one embodiment, the transfer element (116) is provided by a cup-like receptable and an elongated hollow tube for insertion through the access port (120) and the self-sealing cover means (130) into the collection device (110). In this embodiment, the distal end of the connecting element (114a) is shorter than the elongated hollow tube and thus located within the interior of the elongated hollow tube of the transfer element (116). Furthermore, the separation device is comprised in the elongated hollow tube of the transfer element (116) in front of the opening of the distal end of the connecting element (114a). According to a further embodiment, the elongated hollow tube of the transfer element (116) and the connecting element (114) each comprise a separation device. E.g. both the elongated hollow tube of the transfer tube (116) and the connecting element (114) may comprise a filter. According to one embodiment, the pore size of the filters varies. For instance, the pore size of the filter comprised in the elongated hollow tube of the transfer tube (116) is larger than the pore size of the filter comprised in the connecting element (114). Consequently, larger cells are retained in the filter of the transfer element (116) while smaller cells, pathogens, subcellular components and/or further particles are retained in the filter of the connecting element (114). According to one embodiment, the filters can be removed from the elongated hollow tube of the transfer element (116) and the connecting element (114) allowing for separate extraction and analysis of the retained components. Likewise, the separation devices comprised in the elongated hollow tube of the transfer element (116) and the connecting element (114) may be of different types, e.g. one comprises a filter and the other comprises beads. Different suitable combinations are within the scope of the present disclosure.

Optionally, the bottom of the collection device (110) has a reception (112), which can be provided as deepening or heightened rim, into which the distal end of the elongated hollow tube of the transfer element (116) and/or the distal end of the connecting element (114a) extends. This is particularly advantageous to reduce volume losses during sample processing.

It is also within the scope of the present invention that the access port (120) with the cover means (130) for insertion of the transfer device is located in the wall or the bottom of the collection device (110).

### Further embodiments of the invention

**Figures 6** and **7** illustrate further embodiments of the invention wherein urine collection systems are modified to incorporate a separation device that is capable of separating cells from the cell-containing urine sample so that a cell-depleted biological sample is contacted with the stabilizing composition that is comprised in the reception device.

**Figure 6** illustrates an embodiment of the present invention that is based on a commercially available urine collection system that has been modified to include a separation device according to the present invention. **(A)** shows the collection device (urine cup) with collected fluid cell-containing biological sample (here: urine). The collection device is closed with a closing means (lid) that may be screwed onto the collection device. The lid comprises a recess that is dimensioned to receive the evacuated reception device and a transfer device that comprises a transfer element in form of a tube-like hollow protrusion. A connecting element that is provided by a needle is comprised in a coupling element that is mounted to the transfer element. In the shown embodiment the coupling element comprising the connecting element (needle) is inserted into the transfer element and secured by clamp-like fasteners. The coupling element comprises a separation element (e.g. filter - not shown) that is placed before the distal end of the connecting element. The proximal end of the connecting element (needle) reaches into the recess. The reception device comprising the stabilizing composition is provided by an evacuated tube that is pushed into the recess until the cap has been fully penetrated by the connecting element and is hold in position (see **(B)**)**.** The urine is automatically drawn from the collection device into the reception device by the vacuum whereby it is forced through the filter so that contained cells are separated (**(C)**)**.** The cell-depleted biological sample enters the reception device and contacts the stabilizing composition whereby the cell-depleted sample is stabilized. The reception device can then be removed from the recess (**(D)**).

**Figure 7** illustrates a further embodiment that is similar to the embodiment shown in Figure 6. Also here, a coupling element is used that comprises a connecting element (needle) and separation element (filter) that is placed before the distal end of the connecting element so that cells are separated when the fluid cell-containing biological sample (e.g. urine) is transferred through the coupling element. The coupling element is removably attachable to the transfer element (hollow tube) that is mounted to the recess of the lid that receives the reception device. When the coupling element is mounted, the connecting element (needle) reaches into the recess and can thus pierce the cap of the reception device, preferably an evacuated tube, when it is inserted into the recess. In the shown embodiment, the coupling element can be screwed into the transfer element. It therefore can also be removed in an easy manner and the collected cells that are retained in the coupling element can be further processed. The coupling element may also have different shapes and may include e.g. a section with a widened cross-section for receipt of the separation element that is preferably a filter.

The embodiments described in this example 4, in which the transfer device comprising the separation device is permanently incorporated in the lid of the collection device or is removably attachable to the lid (see **Figures 4-7**), provide a more sterile, simpler, faster, and more convenient processing of potentially infectious fluid cell-containing biological samples, such as urine, with increased safety - since no open handling of the sample is required - and reduced effort, in particular since less equipment is needed and less processing steps are required due to simultaneous cell separation and transfer of the sample into the reception device. In addition, contamination and dilution of the target analytes, in particular extracellular nucleic acids (e.g. cfDNA, cfRNA), is prevented or significantly reduced since contaminating components present in the cell-containing biological sample are retained in the separation device. This provides more reliable results. Moreover, this allows separate analysis of the extracellular nucleic acids contained in the cell-depleted biological sample and of the cells and optional further retained components, thereby providing a bigger picture of the patient status.

## Claims

1. A method for stabilizing extracellular nucleic acids contained in a fluid cell-depleted biological sample obtained from a fluid cell-containing biological sample, which preferably is a urine sample, said method comprising
(a) obtaining a fluid cell-containing biological sample in a collection device;
(b) establishing a connection to a reception device which comprises a stabilizing composition suitable for stabilizing extracellular nucleic acids;
(c) transferring the obtained cell-containing biological sample through a separation device, thereby separating cells and providing a cell-depleted biological sample and contacting the cell-depleted biological sample with the stabilizing composition comprised in the reception device, wherein preferably, the transfer is driven by the reception device.

2. The method according to claim 1, wherein the separation device comprises or consists of a separation element that is capable of retaining cells contained in the collected biological sample, and wherein the separation element has one or more of the following characteristics:
(a) the separation element is provided by a filter or beads;
(b) the separation element is porous;
(c) the separation element separates cells and optionally at least one of debris, precipitates and pathogens;
(d) the separation element allows passage of extracellular vesicles;
(e) the separation element comprises functional groups;
(f) the separation element is a porous filter, optionally a membrane, sponge or mesh.

3. The method according to claim 1 or 2, wherein the reception device is an evacuated reception device and wherein in (c) the transfer of the cell-containing biological sample through the separation device is driven by the vacuum of the reception device.

4. The method according to one or more of claims 1 to 3, wherein step (a) comprises closing the collection device comprising the fluid cell-containing biological sample with a closing means, preferably a lid, optionally wherein the closing means comprises a recess adapted to receive the reception device.

5. The method according to one or more of claims 1 to 4, wherein step (b) establishing a connection to the reception device comprises the use of a transfer device that establishes or contributes to establish a connection between the collection device and the reception device wherein said connection enables the transfer of fluid from the collection device to the reception device.

6. The method according to claim 5, wherein the transfer device comprises the separation device or wherein the separation device is attached to the transfer device.

7. The method according to claim 5 or 6, wherein the transfer device is assembled from at least two elements, wherein the transfer device comprises a connecting element, wherein the connecting element has a distal end directed into the collection device and a proximal end directed to the exterior of the collection device, wherein the proximal end preferably comprises a tip, optionally wherein the transfer device has one or more of the following characteristics:
(i) the connecting element is provided by a needle or cannula;
(ii) the transfer device comprises a transfer element that receives the connecting element;
(iii) the connecting element is removably attachable to the transfer element; and/or
(iv) wherein the connecting element is comprised in a coupling element that can be removably attached, preferably screwed or clipped, to the transfer element, optionally wherein the coupling element comprises a separation device;
(v) a separation device for separating cells in step (c) is comprised in at least one element of the transfer device;
(vi) at least two elements of the transfer device comprise a separation device, optionally wherein each separation device is provided by a filter, optionally wherein the pore size of the filters comprised in the different elements differ from each other;
(vii) the transfer element comprises a separation device, optionally wherein the separation device comprises or consists of a filter;
(viii) the connecting element comprises a separation device, optionally wherein the separation device comprises or consists of a filter;

8. The method according to any one of claims 4 to 7, wherein the transfer device or at least an element thereof is connected to the closing means or the collection device, preferably is connected to the closing means which is preferably provided by a lid, optionally wherein the transfer device or at least an element thereof is integrated in or removably attachable to the closing means or the collection device, preferably the closing means which is provided by a lid.

9. The method according to one or more of claims 1 to 8, wherein the stabilizing composition comprised in the reception device protects extracellular nucleic acids comprised in the cell-depleted biological sample from degradation.

10. The method according to claim 9, wherein the stabilizing composition has one or more of the following characteristics:
(i) it comprises a nuclease inhibitor; optionally wherein the nuclease inhibitor is a chelating agent, preferably EDTA;
(ii) it comprises an additive for cell and/or cellular component stabilization, preferably a poly(oxyethylene) polymer, more preferably polyethylene glycol;
(iii) it comprises a high molecular weight poly(oxyethylene) polymer and a low molecular weight poly(oxyethylene) polymer;
(iv) it comprises a preservative for inhibiting bacterial growth;
(v) it comprises a caspase inhibitor;
(vi) it comprises a primary, secondary or tertiary amide; and/or
(vii) it comprises a buffering agent;
(viii) it is a liquid or dried composition.

11. The method according to one or more of claims 1 to 10, wherein the stabilization composition comprised in the reception device is a liquid composition that comprises a chelating agent, preferably EDTA and at least one poly(oxyethylene) polymer, preferably polyethylene glycol, optionally wherein the stabilization composition comprised in the reception device comprises:
- a chelating agent, preferably EDTA, in a concentration of 150mM up to the solubility limit;
- a high molecular weight poly(oxyethylene) polymer, preferably polyethylene glycol, having a molecular weight in the range of 1500 to 40000, such as 3000 to 25000;
- a low molecular weight poly(oxyethylene) polymer, preferably polyethylene glycol, having a molecular weight in the range of 100 to 1000, such as 200 to 700; and
- optionally a buffering agent.

12. The method according to one or more of claims 1 to 11, wherein the collection device comprises a nuclease inhibitor and/or a cell preserving composition, optionally wherein the cell preserving composition has one or more of the following characteristics:
(i) it is a liquid or dried composition, preferably a spray-dried composition;
(ii) the cell preserving composition stabilizes cells and reduces cell lysis;
(iii) it comprises (aa) at least one compound selected from a poly(oxyethylene) polymer, preferably polyethylene glycol (PEG), glycerol, trehalose, dextrans and derivatives thereof, optionally selected from dextran sulfate and dextran acetate, and hydrophilic polymers optionally selected from polyvinyl alcohol, polyvinyl acetate and polyvinyl sulfate and/or (bb) at least one compound selected from sugars, sugar alcohols and salts;
(iv) it comprises a nuclease inhibitor, preferably a chelating agent, more preferably EDTA.

13. The method according to one or more of claims 1 to 12, the method further comprising one or more of the following steps:
(i) isolating extracellular nucleic acids, such as extracellular DNA and/or extracellular RNA, from the stabilized cell-depleted biological sample;
(ii) isolating extracellular vesicles from the stabilized cell-depleted biological sample and optionally isolating nucleic acids, such as RNA, from the isolated extracellular vesicles;
(iii) processing the separated cells, optionally wherein processing the separated cells comprises (i) analysing the separated cells and/or (ii) extracting nucleic acids from the separated cells.

14. A method for isolating extracellular nucleic acids from a stabilized cell-depleted biological sample, the method comprising
- stabilizing extracellular nucleic acids in a cell-depleted biological sample obtained from a fluid cell-containing biological sample using the method according to claims 1 to 13; and
- isolating extracellular nucleic acids from the stabilized cell-depleted biological sample.

15. The method according to claim 14, wherein the cell-containing biological sample is a urine sample and wherein the extracellular nucleic acid is extracellular DNA.

16. A kit for stabilizing extracellular nucleic acids in a cell-depleted biological sample obtained from a fluid cell-containing biological sample, which preferably is a urine sample, said kit comprising
(a) a collection device;
(b) a reception device comprising a stabilizing composition suitable for stabilizing extracellular nucleic acids comprised in the cell-depleted biological sample; and
(c) a separation device that comprises or consists of a separation element that is capable of retaining cells contained in the collected biological sample.

17. The kit according to claim 16, wherein the kit further comprises
(d) a closing means for closing the collection device, optionally wherein the closing means has the characteristics as defined in claim 4; and/or
(e) a transfer device for assisting the transfer of fluids from the collection device into the reception device, optionally wherein the transfer device has the characteristics as defined in one or more of claims 5 to 8.

18. The kit according to claim 16 or 17, wherein the kit has one or more of the following characteristics:
(i) the reception device has the characteristics as defined in claim 3;
(ii) the separation device has the characteristics as defined in claim 2;
(iii) the stabilizing composition comprised in the reception device has the characteristics as defined in any one of claims 9 to 11;
(iv) the collection device has the characteristics as defined in claim 12 and/ or comprises a closing means.

19. Use of a kit according to one or more of claims 16 to 18 in a method according to one or more of claims 1 to 15.

20. Use of a separation device that comprises or consists of a separation element that is capable of retaining cells in a method for stabilizing extracellular nucleic acids contained in a fluid cell-depleted biological sample obtained from a fluid cell-containing biological sample, wherein stabilization of the cell-depleted biological sample is performed in a reception device that comprises a stabilizing composition, and wherein said method comprises transferring the cell-containing biological sample through the separation device, thereby separating cells and providing a cell-depleted biological sample and contacting the provided cell-depleted biological sample with the stabilizing composition comprised in the reception device, wherein the transfer is driven by the reception device.
